# EUROPEAN PATENT APPLICATION

(11) **EP 4 609 790 A1**
(43) Date of publication of application: **03.09.2025**
(21) Application number: 23882556.6
(22) Date of filing: 20.10.2023
(51) Int. Cl.: A61B 5/352, A61B 5/00, A61B 5/11, A61B 5/1455

(54) **INFORMATION PROCESSING DEVICE, INFORMATION PROCESSING METHOD, AND PROGRAM**

(30) Priority: 24.10.2022 JP 2022169965
(71) Applicant: Suntory Holdings Limited, Osaka 530-8203 (JP)
(72) Inventor: MIZUTANI Haruo, Kyoto 6190284 (JP)
(74) Representative: Vossius & Partner Patentanwälte Rechtsanwälte mbB
(86) International application number: PCT/JP2023/038001
(87) International publication number: WO 2024/090351

(57) **Abstract**

In order to solve the problem that it is difficult to acquire a score indicating a physical condition of a user, an information processing apparatus 200 includes: a biological information acquiring unit 143 that acquires measurement information on a status of a living body, based on measurement results of a bioelectric potential of a user at two or more points in time; and a score acquiring unit 251 that acquires score information containing a score indicating a present or future physical condition of the user, based on the measurement information. Accordingly, it is possible to acquire a score indicating a present or future physical condition of an individual user.

## Description

### Technical Field

The present invention relates to an information processing apparatus, an information processing method, and a program that are capable of acquiring information on a present or future physical condition of a user.

### Background Art

In recent years, users have become increasingly aware of their own health, and various devices and services have been provided to assist users in understanding their health status. For example, Patent Document 1 below discloses a system configured to determine a health risk signal of a user based on an aggregate heartbeat value determined according to biological measurement data obtained from the user, and to provide the signal to the user.

Furthermore, Patent Document 2 discloses a relaxation level determination apparatus that can easily and accurately determine the relaxation level by using the heartbeat (pulse) signal of an examinee who is a user.

It is known that various rhythms affect human sleeping and waking. Such rhythms include, for example, a rhythm called circadian rhythm (circadian cycle). The so-called two-process rhythm is also widely known as the human sleep-wake rhythm (see Non-Patent Document 1 below, for example).

Furthermore, in addition to the conventionally known Transformer, a method called Informer has been proposed as a machine learning method that can be used for prediction of future time-series data, as described in Non-Patent Document 2 below.

### Citation List

### Patent Documents

Patent Document 1: Japanese Patent No. 6531161
Patent Document 2: JP H9-70399A

### Non-Patent Documents

Non-Patent Document 1: Daan S, Beersma DG, Borbely AA. Timing of human sleep: recovery process gated by a circadian pacemaker. Am J Physiol. 1984; 246 (2 Pt 2): R161-83.
Non-Patent Document 2: Zhou, Haoyi, et al. "Informer: Beyond efficient transformer for long sequence time series forecasting." Proceedings of the AAAI Conference on Artificial Intelligence. Vol. 35. No. 12.

### Summary of Invention

### Technical Problem

In order to maintain users' health, it is effective to enable users to easily understand and evaluate their own health status. To enable users to understand their own health status, it is considered useful to use scores that indicate the users' physical conditions. However, conventionally, it has been difficult to obtain such scores.

It is an object of the present invention to provide an information processing apparatus, an information processing method, and a program that are capable of acquiring a score indicating a present or future physical condition of an individual user.

### Solution to Problem

A first aspect of the present invention is directed to an information processing apparatus including: a biological information acquiring unit that acquires measurement information on a status of a living body, based on measurement results of a bioelectric potential of a user at two or more points in time; and a score acquiring unit that acquires score information containing a score indicating a present or future physical condition of the user, based on the measurement information.

With this configuration, it is possible to acquire a score indicating a present or future physical condition of an individual user.

Furthermore, a second aspect of the present invention is directed to the information processing apparatus according to the first aspect, wherein the measurement information is time-series information.

With this configuration, it is possible to acquire score information more accurately indicating a physical condition.

Furthermore, a third aspect of the present invention is directed to the information processing apparatus according to the first or second aspect, wherein the score information contains information indicating transition of the score.

With this configuration, it is possible to acquire information indicating transition of a score of an individual user.

Furthermore, a fourth aspect of the present invention is directed to the information processing apparatus according to any one of the first to third aspects, further including: an output information acquiring unit that acquires output information on the score based on the score information; and an output unit that outputs the output information.

With this configuration, it is possible to provide a user with information corresponding to a present or future physical condition.

Furthermore, a fifth aspect of the present invention is directed to the information processing apparatus according to the fourth aspect, wherein the output information acquiring unit acquires a largest value of a score of score information that may be acquired for the user, and acquires output information corresponding to a relationship between the largest value and the score information acquired by the score acquiring unit.

With this configuration, it is possible to provide a user with information corresponding to a relationship between a status in which a score has the largest value and a present or future physical condition.

Furthermore, a sixth aspect of the present invention is directed to the information processing apparatus according to the fourth aspect, wherein the measurement information is information obtained by measuring a bioelectric potential of a user using a measuring device having two or more electrodes that are in contact with a body surface of the user.

With this configuration, it is possible to easily obtain measurement results of a bioelectric potential of a user.

Furthermore, a seventh aspect of the present invention is directed to the information processing apparatus according to the sixth aspect, wherein the output information acquiring unit acquires output information for outputting a sound from the measuring device.

With this configuration, it is possible to inform a user of information on the score by causing the measuring device to output a sound.

Furthermore, an eighth aspect of the present invention is directed to the information processing apparatus according to any one of the fourth to seventh aspects, wherein the output information acquiring unit acquires, as the output information, advice on eating-and-drinking behavior of a user to ensure that a score higher than the score of the score information acquired by the score acquiring unit is obtained in the future.

With this configuration, it is possible to inform a user of advice on eating-and-drinking behavior that will help him or her achieve better conditions in the future.

Furthermore, a ninth aspect of the present invention is directed to the information processing apparatus according to any one of the first to eighth aspects, wherein the score acquiring unit acquires the score information, based on information obtained by measuring at least one of a blood pressure, a pulse, a blood oxygen level, a body temperature, a respiratory rate, and an acceleration of a user.

With this configuration, it is possible to acquire score information that accurately reflects a physical condition of a user.

Furthermore, a tenth aspect of the present invention is directed to the information processing apparatus according to any one of the first to ninth aspects, wherein the score acquiring unit acquires the score information, based on eating-and-drinking information on eating-and-drinking behavior of a user.

With this configuration, it is possible to acquire score information that accurately reflects a physical condition of a user.

Furthermore, an eleventh aspect of the present invention is directed to the information processing apparatus according to any one of the first to tenth aspects, wherein the score acquiring unit acquires future measurement information, based on input information on the measurement information, using learning information configured such that the input information is taken as input and the future measurement information is taken as output, and acquires the score information based on the acquired measurement information.

With this configuration, it is possible to acquire score information that accurately reflects a physical condition of a user.

Furthermore, a twelfth aspect of the present invention is directed to the information processing apparatus according to any one of the first to tenth aspects, wherein the score acquiring unit acquires information on the score, based on input information on the measurement information, using learning information configured such that the input information is taken as input and the information on the score is taken as output, and acquires the score information using the acquired information.

With this configuration, it is possible to accurately predict a score that reflects a future physical condition of a user.

Furthermore, a thirteenth aspect of the present invention is directed to the information processing apparatus according to the twelfth aspect, wherein the score acquiring unit uses, as the learning information, learning information configured using input information containing measurement information obtained by measuring two or more users in the past.

With this configuration, it is possible to acquire accurate score information even if there is little measurement information on a user himself or herself.

Furthermore, a fourteenth aspect of the present invention is directed to the information processing apparatus according to the twelfth aspect, further including a learning information acquiring unit that acquires learning information obtained by performing re-learning using evaluation information corresponding to the score information acquired by the score acquiring unit and input information used to acquire the score information.

With this configuration, it is possible to increase the accuracy of acquired score information in accordance with the score information being continuously acquired.

### Advantageous Effects of Invention

With the information processing apparatus and the like according to the present invention, it is possible to acquire a score indicating a present or future physical condition of an individual user.

### Brief Description of Drawings

FIG. 1 is a diagram showing the outline of an information processing system according to Embodiment 1 of the present invention.
FIG. 2 is a block diagram of an information processing apparatus in the embodiment.
FIG. 3 is a block diagram of a terminal device and a measuring device in the embodiment.
FIG. 4 is a diagram illustrating the structure of learning information that is used in the information processing apparatus in the embodiment.
FIG. 5 is a flowchart showing an example of an operation of the information processing apparatus in the embodiment.
FIG. 6 is a flowchart showing an example of prediction information acquiring processing of the information processing apparatus in the embodiment.
FIG. 7 is a diagram illustrating time-series information in a specific example of this embodiment.
FIG. 8 is a diagram showing an example of time-series information before filter processing in a specific example of this embodiment.
FIG. 9 is a diagram showing an example of time-series information after filter processing in a specific example of this embodiment.
FIG. 10 is a diagram showing an example of prediction information acquired in a specific example of this embodiment.
FIG. 11 is a diagram showing the outline of the information processing system according to Embodiment 2 of the present invention.
FIG. 12 is a block diagram of the information processing apparatus in the embodiment.
FIG. 13 is a flowchart showing an example of an operation of the information processing apparatus in the embodiment.
FIG. 14 is a flowchart showing an example of score information acquiring processing of the information processing apparatus in the embodiment.
FIG. 15 is a flowchart showing an example of life score acquiring processing of the information processing apparatus in the embodiment.
FIG. 16 is a first diagram showing a specific example of screen transition of the terminal device in the embodiment.
FIG. 17 is a second diagram showing a specific example of screen transition of the terminal device in the embodiment.
FIG. 18 is a schematic view of a computer system in the foregoing embodiments.
FIG. 19 is a block diagram of the computer system in the embodiments.

### Description of Embodiments

Hereinafter, embodiments of an information processing apparatus and the like will be described with reference to the drawings. It should be noted that constituent elements denoted by the same reference numerals in the embodiments perform similar operations, and thus a description thereof may not be repeated.

The terms used below are generally defined as follows. The meanings of these terms do not always have to be interpreted as indicated herein, and have to be interpreted in light of individual explanations below, if any, for example.

"Identifier for a matter" is a letter, a symbol, or the like for uniquely identifying the matter. The identifier is an ID, for example, but may be any type of information with which the corresponding matter can be identified. That is to say, the identifier may be the name of the matter itself that it indicates, or symbols that are combined so as to uniquely correspond to the matter.

"Acquiring" may include acquiring a matter that is input by a user or the like, or acquiring information stored in the apparatus or another apparatus (the information may be information stored in advance or information generated through information processing in the apparatus). "Acquiring information stored in another apparatus" may include acquiring information stored in the other apparatus via an API or the like, or acquiring the content of a document file (including the webpage content, etc.) provided by the other apparatus.

Furthermore, a so-called machine learning method may be used to acquire information. A machine learning method may be used as follows, for example. That is to say, a learning model (learning information) in which a particular type of input information is taken as input and a type of output information that is to be acquired is taken as output is configured using a machine learning method. For example, two or more pairs of input information and output information are prepared in advance, the two or more pairs of information are given to a module for configuring a learning model of machine learning to configure a learning model, and the configured learning model is accumulated in a storage unit. The learning model may also be said to be a classifier. There is no limitation on the machine learning method, and examples thereof include deep learning, random forests, and SVM. For example, functions in various machine learning frameworks and various existing libraries, such as fastText, tinySVM, random forest, and TensorFlow, can be used for the machine learning. The acquiring information using such a learning model may also be said to be acquiring information through machine learning.

"Learning model" is not limited to those obtained through machine learning. The learning model may be a table indicating a correspondence relationship between an input vector based on input information or the like and output information, for example. In this case, output information corresponding to a feature vector based on input information may be acquired from the table, or a vector that is close to a feature vector based on the input information may be generated using two or more input vectors in the table and parameters for weighting the input vectors, and output information corresponding to the input vectors and parameters used for the generation may be used to acquire final output information. The acquiring information using such a learning model may also be said to be acquiring information using a correspondence relationship. The learning model may be a function or the like that represents a relationship between an input vector based on the input information or the like and information for generating output information, for example. In this case, for example, information corresponding to a feature vector based on input information may be obtained using a function, and the obtained information may be used to acquire output information. The acquiring information using such a learning model may also be said to be acquiring information using a function.

"Outputting information" is a concept that encompasses display on a display screen, projection using a projector, printing by a printer, output of a sound, transmission to an external apparatus, accumulation in a recording medium, and delivery of a processing result to another processing apparatus or another program. Specifically, for example, this concept encompasses enabling information to be displayed on a webpage, transmission as an email or the like, and outputting information for printing.

"Accepting information" is a concept that encompasses accepting information input from an input device such as a keyboard, a mouse, or a touch panel, receiving information transmitted from another apparatus or the like via a wired or wireless communication line, and accepting information read from a recording medium such as an optical disk, a magnetic disk, or a semiconductor memory.

### Embodiment 1

In this embodiment, an information processing system is configured using an information processing apparatus. The information processing apparatus is configured to acquire prediction information on a future biological signal of a user who is a provider, from measurement results of a biological signal of the user, and to output future information on a future status of the user based on the prediction information. The information processing apparatus may be called a prediction information output apparatus, and the information processing system may be called a prediction information output system.

Preferably, the information processing apparatus may be configured as follows. For example, the measurement results may be time-series information, and prediction information may be acquired from time-series information after predetermined pre-processing. For example, the pre-processing may be singular spectrum analysis, and time-series information after the processing may be configured based on the results having degrees up to a predetermined highest degree obtained through singular spectrum analysis. For example, the prediction information may be acquired using a neural network model, and in particular, a Transformer model may be used. In this case, the decoders of the Transformer model may be configured not to perform autoregression.

As a specific example, the information processing apparatus may be configured, for example, to use information indicating the transition of LF, HF, or LF/HF based on an electrocardiogram (ECG), as the time-series information. For example, a time period during which the user is likely to feel stressed or a time period during which the user is likely to be relaxed in a predetermined period of time may be specified from the prediction information, and information indicating that time period may be output.

Hereinafter, an example of the information processing system using the thus configured information processing apparatus will be described.

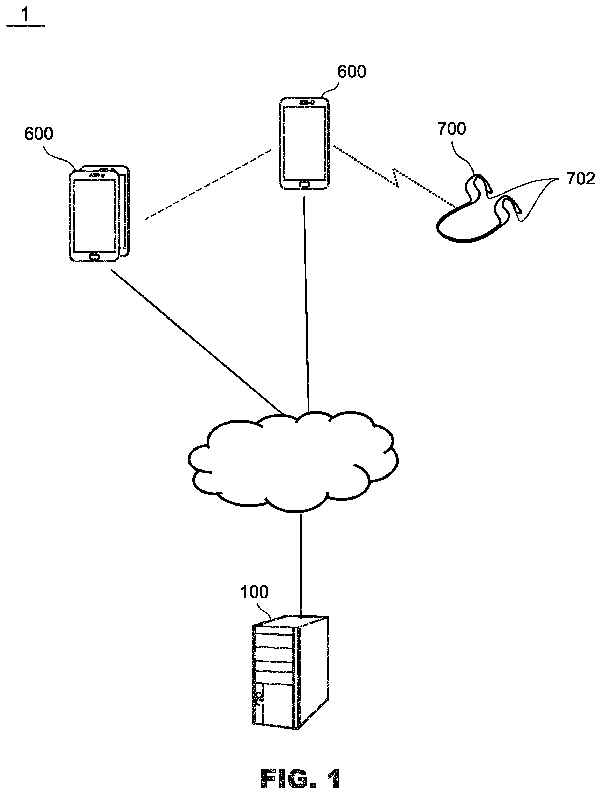

FIG. 1 is a diagram showing the outline of an information processing system 1 according to Embodiment 1 of the present invention.

In this embodiment, the information processing system 1 includes an information processing apparatus 100, terminal devices 600, and measuring devices 700. The information processing apparatus 100 and the terminal devices 600 can communicate with each other, for example, via a network such as a local area network or the Internet. A measuring device 700 and a terminal device 600 can communicate with each other. The configuration of the information processing system 1 is not limited to this. There is no limitation on the number of apparatuses of each type included in the information processing system 1, and other types of apparatuses may also be included in the information processing system 1.

Each measuring device 700 is, for example, a so-called wearable device. The measuring device 700 has electrode units 702 that are in contact with a body surface of the user. The measuring device 700 is configured to be able to measure biological signals related to bioelectricity such as action potentials and resting potentials of the body of the user via the electrode units 702. In this embodiment, the measuring device 700 is configured to be able to measure time-series biological signals such as brain waves and pulse waves.

The measuring device 700 may be configured to be able to measure the body temperature or the like of the user by means of the electrode units 702 or other unshown units. The measuring device 700 may include a measuring unit for measuring biological signals of the user instead of the electrode units 702 or in addition to the electrode units 702. The measuring unit may be configured to be able to detect a target different from the bioelectricity. For example, the measuring unit may be able to receive light emitted or reflected from the body of the user to obtain time-series measurement results. That is to say, the measuring device 700 may be configured, for example, to be able to receive light emitted or reflected from the body of the user and to measure information on the body of the user based on the results thereof. The measuring device 700 includes, for example, a storage unit (not shown) in which measurement results, control programs for the measuring device 700, and the like are stored, a processing unit (not shown) that performs various processing operations using the information stored in the storage unit, a transmitting and receiving unit (not shown) for transmitting and receiving information, and the like.

The measuring device 700 is, for example, an ear-mounted headset that can be worn by the user at all times. The measuring device 700 is configured such that, when worn, two electrode units 702 are in contact with the vicinity of the left and right mastoid processes of the user, thereby enabling measurement of the brain waves and the pulse waves of the user. Such a configuration of the measuring device 700 is known, and thus a detailed description thereof has been omitted. The measuring device 700 is not limited to a headset type, and may be in various forms that allow the user to continue wearing it, such as eyeglasses, wristwatch, ring, neck-hanging, belt, and clothing types. The measuring device 700 may include a sound output unit (not shown) that outputs a user-hearable sound by vibrating air or an object, a display screen (not shown) that can be viewed by the user, and the like. The measuring device 700 may be configured to be able to output information to the user by means of the sound output unit, the display screen, and the like. The measuring device 700 may be two or more physically separated apparatuses that can cooperate to measure one or more biological signals. The measuring device 700 may include electrode units 702 that are used in contact with the body surface of the user only during the measurement. The measuring device 700 is not limited to such wearable devices, but may also be those that are used by the user to measure biological signals under certain circumstances, such as, for example, a blood pressure monitor, a thermometer, a scale, an electroencephalograph, or an electrocardiograph.

In this embodiment, the measurement results of time-series biological signals and information based thereon are referred to as measurement information on a status of a living body. The measurement information may be, for example, raw data of measurement results acquired by measuring biological signals, or information obtained by converting or processing the raw data. The measurement information may also be said to be time-series information that is time-series information on a status of a living body based on measurement results of a biological signal of a user. That is to say, time-series information is measurement results of time-series biological signals, or time-series information acquired based thereon.

In this embodiment, examples of the measurement results of a biological signal include, but are not limited to, ECG (electrocardiogram), pulse waves such as PPG (photoplethysmogram), brain waves, and the like.

The measuring device 700 in this embodiment is connected wirelessly or via a wired connection to the terminal device 600, and is configured to be able to transmit and receive information to and from the terminal device 600. For example, the measuring device 700 is configured to be able to perform short-range wireless communication with the terminal device 600. The measuring device 700 is configured, for example, to be able to transmit the measurement results to the terminal device 600. The measurement results can be transmitted to the terminal device 600 as time-series information accumulated by the measuring device 700, or can be transmitted sequentially to the terminal device 600. The terminal device 600 is configured to be able to perform various types of processing using information received from the measuring device 700, and to transmit the received information or information obtained by processing the received information to the information processing apparatus 100. The measuring device 700 itself may be able to be connected to a network such as a local area network or the Internet, and to communicate with the information processing apparatus 100 and the terminal device 600 connected to the network. The measurement results may be transmitted to the information processing apparatus 100. The measuring device 700 may be configured to be able to perform operations such as measuring biological signals or performing predetermined processing on measurement results by itself, or may be configured to be able to perform these operations in conjunction with the terminal device 600 by transmitting and receiving signals.

The users of the information processing system 1 can use the information processing system 1 by using the terminal devices 600 and the measuring devices 700. In FIG. 1, for example, portable information terminal devices such as so-called smartphones are shown as the terminal devices 600, but those that are used as the terminal devices 600 are not limited to such portable information terminal devices. For example, personal computers (PC) such as laptop computers may be used as the terminal devices 600, or other devices such as tablet-type information terminal devices may be used. In the examples below, a description will be given assuming that portable information terminal devices such as smartphones are used as the terminal devices 600, but there is no limitation to this.

The terminal devices 600 may include built-in measuring devices 700.

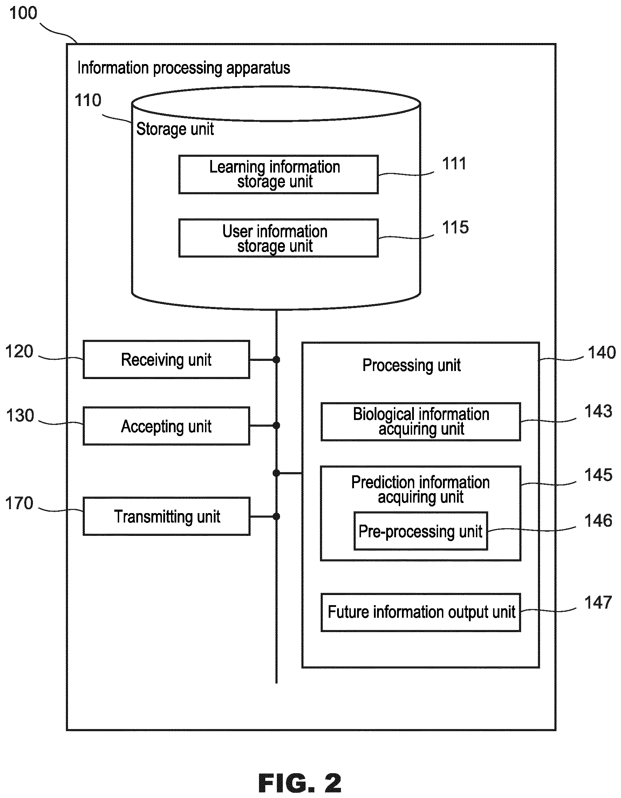

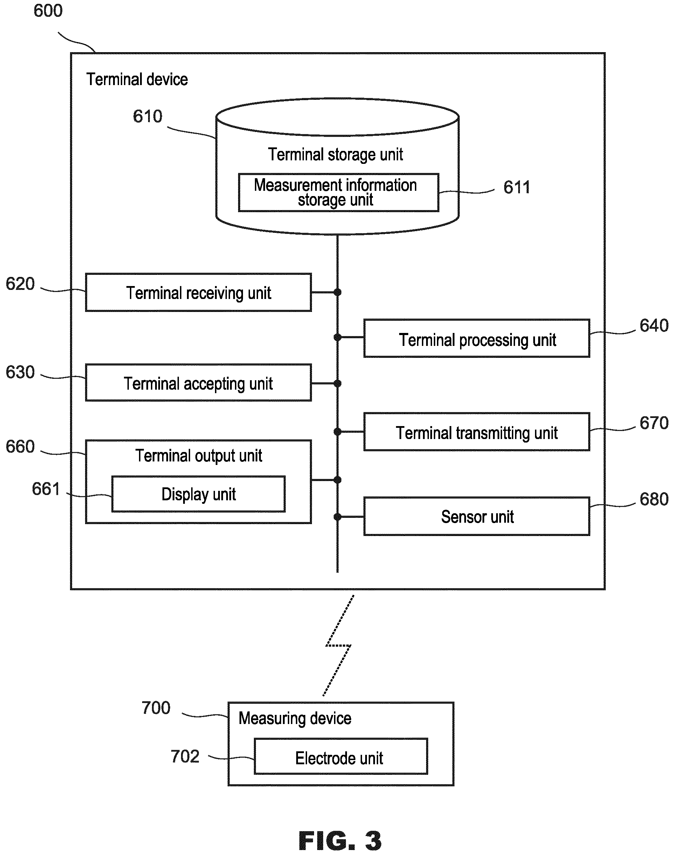

FIG. 2 is a block diagram of the information processing apparatus 100. FIG. 3 is a block diagram of a terminal device 600 and a measuring device 700.

As shown in FIG. 2, the information processing apparatus 100 includes a storage unit 110, a receiving unit 120, an accepting unit 130, a processing unit 140, and a transmitting unit 170. The information processing apparatus 100 is, for example, a server apparatus.

The storage unit 110 includes a learning information storage unit 111 and a user information storage unit 115.

Learning information acquired in advance is stored in the learning information storage unit 111. In this embodiment, the learning information is generated using a so-called machine learning method. The learning information is, for example, generated and stored in the learning information storage unit 111 by the processing unit 140 as described later, but there is no limitation to this. That is to say, learning information generated in an apparatus different from the information processing apparatus 100 may be stored in the learning information storage unit 111. Details of the learning information will be described later.

In this embodiment, the learning information is configured for each user targeted for biological signal measurement. Learning information corresponding to each user is generated and stored in the learning information storage unit 111. Each piece of learning information is stored in association with a user identifier for identifying a user. However, there is no limitation to this, and learning information that can be commonly used for two or more users may be prepared. Learning information may be prepared for each group of users who share common attributes. In this case, for example, learning information may be stored in association with an identifier for identifying a group.

User information is stored in the user information storage unit 115. In this embodiment, the user information is information in which a user identifier, which is an identifier for identifying a user who uses the information processing system 1, is associated with information on the user. The user information may contain various types of information. For example, the user information may contain information transmitted from the terminal device 600 that is used by the user, information on the user acquired by the information processing apparatus 100 as described later, and the like. The information transmitted from the terminal device 600 that is used by the user corresponds to, for example, time-series information, information input by the user to the terminal device 600, and the like. The information on the user acquired by the information processing apparatus 100 corresponds to, for example, information on the sleep-wake rhythm of the user and prediction information based thereon, as will be described later, and the like. User information transmitted from other external apparatuses or the like may be stored in the user information storage unit 115.

Time-series information transmitted from the terminal devices 600 of the respective users is stored in the user information storage unit 115. The time-series information is, for example, measurement results obtained by the users performing measurement using the measuring devices 700, and is information stored in terminal storage units 610 of the terminal devices 600. The procedure in which the time-series information is stored in the user information storage unit 115 is not limited to this. The configuration is also possible in which time-series information prepared in advance is stored in the user information storage unit 115 and that time-series information is used.

The receiving unit 120 receives information transmitted from other apparatuses. The receiving unit 120 stores the received information, for example, in the storage unit 110. In this embodiment, for example, the users perform input of information and the like and transmit the information to the information processing apparatus 100, using the terminal devices 600. The receiving unit 120 can store each piece of transmitted information in the storage unit 110 in association with the user identifier. In this embodiment, the receiving unit 120 receives the time-series information transmitted from the terminal devices 600, and stores the information in the storage unit 110 in association with the user identifiers. In the case in which the receiving unit 120 receives these pieces of information from the terminal devices 600, the receiving unit 120 can specify user identifiers of the users pertaining to the transmission based on the transmitted information.

The accepting unit 130 accepts information input using an unshown input part connected to the information processing apparatus 100. The accepting unit 130 stores the accepted information, for example, in the storage unit 110. The information may be input by any input part such as a numeric keypad, a keyboard, a mouse, or a menu screen. The accepting unit 130 may accept information input through an input operation performed using a reading device (e.g., code reader, etc.) connected to the information processing apparatus 100 (e.g., including information read by the device).

The accepting unit 130 may be considered to accept information received by the receiving unit 120, as information input to the information processing apparatus 100. That is to say, the input of information to the information processing apparatus 100 may be construed to mean that the information is indirectly input to the information processing apparatus 100 by the user via the terminal device 600 or the like or that the information is directly input to the information processing apparatus 100 by the user using an input part. The processing in which the user causes the information processing apparatus 100 to execute a program for automatically generating information or to function by giving various types of information to a program, thereby causing information to be given to the information processing apparatus 100 may be considered as the input of information to the information processing apparatus 100.

The processing unit 140 includes a biological information acquiring unit 143, a prediction information acquiring unit 145, and a future information output unit 147. In this embodiment, the prediction information acquiring unit 145 includes a pre-processing unit 146. The processing unit 140 performs various types of processing. The various types of processing are, for example, processing that is performed by the units of the processing unit 140 as follows.

The biological information acquiring unit 143 acquires time-series information for a user targeted for prediction. In this embodiment, the biological information acquiring unit 143 acquires the time-series information transmitted from the terminal device 600 of the user and received by the receiving unit 120, from the user information storage unit 115. The biological information acquiring unit 143 acquires time-series information on a user targeted for processing, from the user information storage unit 115, based on the user identifier of the user.

The prediction information acquiring unit 145 acquires prediction information on future time-series information on a user, using time-series information on the user and learning information corresponding to the user. The acquired prediction information is, for example, stored in the storage unit 110. The term "future" refers to, for example, a period or point in time later than the end of the period of time-series information acquired for a user targeted for prediction, and in particular refers to a period or point in time that includes a point in time later than the point in time when the prediction information is acquired.

The prediction information is acquired using a machine learning method. That is to say, in this embodiment, the learning information is configured using a machine learning method using time-series information acquired from measurement results of a biological signal in the past (which may also be said to be past time-series information). The past time-series information may be information on time-series measurement results themselves, or information obtained using the time-series measurement results. The prediction information is information obtained by predicting information that temporally follows the past time-series information. The prediction information may also be said to be prediction results of time-series information in a predetermined period of time later than the end of the period of past time-series information.

The learning information is information that is used to configure a learning model in which past time-series information is taken as input information and prediction information on future time-series information is taken as output information. For example, the processing unit 140 can generate the learning information as follows, for example. That is to say, the processing unit 140 configures, using a machine learning method, learning information in which past time-series information is taken as input and prediction information on future time-series information is taken as output. For example, two or more pairs of past time-series information and output values, for learning, are prepared in advance, learning information is configured by giving the two or more pairs of information to a module for configuring learning information of machine learning, and the configured learning information is accumulated in the learning information storage unit 111. Examples of the machine learning method include, but are not limited to, those using recursive neural networks (RNN) such as long- and short-term memory. For example, functions in various machine learning frameworks and various existing libraries, such as TensorFlow and PyTorch, can be used for the machine learning. The pairs of information for learning may be prepared using results obtained by measuring each user in advance. Such learning information may be generated by an apparatus different from the information processing apparatus 100.

In this embodiment, the prediction information acquiring unit 145 is configured to acquire the prediction information using the time-series information after the processing by the pre-processing unit 146. That is to say, the learning information as described above is generated using, as input, past time-series information after the processing. Examples of the processing by the pre-processing unit 146 include, but are not limited to, those described later.

The pre-processing unit 146 performs predetermined filter processing on time-series information acquired by the biological information acquiring unit 143 (referred to as pre-processing information), thereby acquiring time-series information after the processing. In other words, the pre-processing unit 146 performs dimensionality reduction (dimensionality compression) on the pre-processing information. The pre-processing unit 146 is configured, for example, to perform known singular spectrum analysis, as the predetermined filter processing. Accordingly, prediction information can be acquired using time-series information after the processing in which oscillating and trend components are extracted. Such filter processing can be performed, for example, using a numerical calculation library such as NumPy, for example.

The pre-processing unit 146 is configured to acquire time-series information after the processing, using results having degrees up to a predetermined highest degree obtained through singular spectrum analysis. The predetermined degree is, for example, 1 or higher and 10 or lower, and preferably 5 or higher and 10 or lower. In this embodiment, the pre-processing unit 146 is configured to acquire time-series information after the processing, using the results having degrees up to a highest degree of 10 obtained through singular spectrum analysis.

The method by which the pre-processing unit 146 performs dimensionality reduction is not limited to this. For example, the pre-processing unit 146 may be configured to perform dimensionality reduction of pre-processing information using an autoencoder using a convolutional neural network. The dimensionality reduction using an autoencoder can be configured using known methods. For example, the pre-processing information may be input to a probabilistic encoder to extract vectors indicating latent oscillating components and vectors indicating latent trend components, respectively.

The future information output unit 147 outputs future information on a future status of a user targeted for prediction, based on the prediction information. The future information may be, for example, prediction information itself, or information acquired based on the prediction information. Examples of the information acquired based on the prediction information may include, but are not limited to, a condition or status of the user at a future point in time or during a future period of time, expressed in a predetermined classification, and a score. For example, the information may be information on comparison results obtained through comparison between a reference value of each user obtained from past measurement information and prediction information, or information on comparison results obtained through comparison between a predetermined reference value commonly used for multiple users and prediction information.

In this embodiment, the future information output unit 147 may be configured to acquire, as the future information, information on a time period during which a user is likely to feel stressed or a time period during which the user is likely to be relaxed in a predetermined period of time, based on the prediction information, and to output the acquired future information. Examples of the predetermined period of time include, but are not limited to, the period of the prediction information. For example, the future information output unit 147 specifies a time period that satisfies a predetermined determination condition based on the prediction information, and acquires information on the specified time period, as future information. For example, a case will be assumed in which the prediction information relates to time-series information indicating sympathetic and parasympathetic activities, respectively. Examples of such prediction information include, but are not limited to, information on LF and HF related to heartbeat variability and information on brain wave activity. In such cases, for example, the predetermined determination condition can be set to be a state in which either sympathetic or parasympathetic activity is dominant over the other. Also, for example, the predetermined determination condition can be set to be a state in which the degree of dominance of either sympathetic or parasympathetic activity over the other is greater than or equal to a predetermined degree.

The output of future information is performed, for example, by transmitting information from the transmitting unit 170 to the terminal device 600 of the user, but there is no limitation to this. For example, the output of future information may be a hand-off to processing that is performed by the information processing apparatus 100. For example, the output may be performed by displaying the prediction information in the form of text or images on a display screen provided by the information processing apparatus 100. The output of future information may also mean accumulating the future information in the storage unit 110 to perform these processes.

The transmitting unit 170 transmits information to other devices constituting the information processing system 1 via a network. The transmitting unit 170 transmits information, for example, to the terminal device 600. In other words, the transmitting unit 170 outputs information, for example, to the terminal device 600.

Next, the configuration of a terminal device 600 will be described.

As shown in FIG. 3, the terminal device 600 includes a terminal storage unit 610, a terminal receiving unit 620, a terminal accepting unit 630, a terminal processing unit 640, a terminal output unit 660, a terminal transmitting unit 670, and a sensor unit 680. The terminal output unit 660 includes a display unit 661.

Various types of information are accumulated in the terminal storage unit 610. The terminal storage unit 610 includes a measurement information storage unit 611. For example, measurement information measured by the measuring device 700 is accumulated in the measurement information storage unit 611. Also, future information transmitted from the information processing apparatus 100 is accumulated in the terminal storage unit 610.

The terminal receiving unit 620 receives information transmitted from the information processing apparatus 100, the measuring device 700, and the like, via a network. The terminal receiving unit 620 accumulates the received information, for example, in terminal storage unit 610 such that the information can be acquired by the terminal processing unit 640 and the like.

The terminal accepting unit 630 accepts various input operations performed on the terminal device 600 by a user who uses the terminal device 600. The operations are performed using, for example, an unshown input device, but there is no limitation to this.

The terminal processing unit 640 performs various information processing operations using the units of the terminal device 600. For example, future information transmitted from the information processing apparatus 100 is output by the terminal output unit 660 to the user. This allows the user to learn about the future information.

The terminal output unit 660 outputs information by, for example, displaying it on the display unit 661 that is a display device. The method for outputting information is not limited to this, and may also be performed by outputting a sound or the like from a speaker or the like.

The terminal transmitting unit 670 transmits information acquired by the terminal processing unit 640 or the like, for example, via a network.

Examples of the sensor unit 680 include, but are not limited to, a microphone, an acceleration sensor, a barometric pressure sensor, and the like. The sensor unit 680 may be used to measure a biological signal of the user. The sensor unit 680 may include electrodes that can measure a biological signal of the user.

In this case, machine learning methods that may be used in this embodiment will be described.

As learning information, information is used that can constitute neural network models with encoders and decoders using so-called Transformer. Such neural network models called Transformer are known models used to predict time-series information, but may be partially modified. Transformer-based neural network models may be said to have the encoder-decoder structure with a self-attention mechanism. Transformer-based neural network models may be said to have the encoder-decoder structure using a multi-head attention mechanism. In this embodiment, the learning information is a neural network model that uses, in part, the so-called Informer method as described in Non-Patent Document 2 above. Informer may be said to be a derivative neural network model of Transformer. The decoders of the neural network model that is constituted by the learning information are configured not to perform autoregression. That is to say, the learning information is a neural network model that uses the so-called Informer method in part with a generative-style decoder structure that does not involve autoregression, and that constitutes a model with encoders and decoders using standard Transformer. The learning information can constitute a neural network model with encoders and decoders in which ProbSparse Self Attention and Encoder-distilling methods are not used among the methods called Informer.

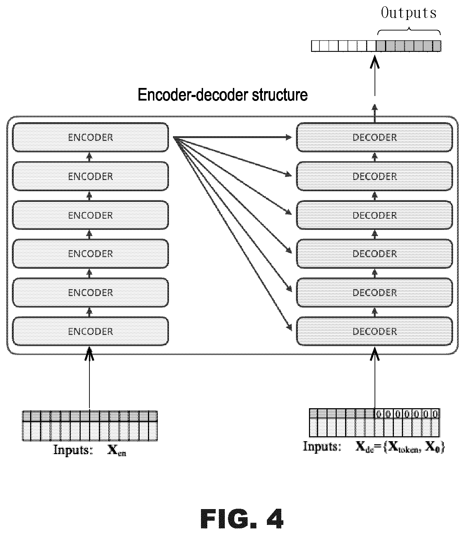

FIG. 4 is a diagram illustrating the structure of learning information that is used in the information processing apparatus 100.

The drawing schematically shows the encoder-decoder structure of the learning information. Generative-style decoders can be described as follows. That is to say, in standard Transformer, the input data corresponding to the sequence of data output by the decoders is missing in the last part and is shifted backward as a whole, and only one first piece of data is padded. In contrast, in generative-style decoders, as shown in the drawing, all the portions of the input data corresponding to the sequence of data to be output are padded in the inference unit, so that the output data portion is generated at once during inference.

With this configuration using generative-style decoders of Informer, it is possible to acquire prediction information at higher speed.

Next, an example of the operation of the information processing apparatus 100 performed when a user uses the information processing system 1 according to this embodiment will be described. In this embodiment, the user can use the information processing system 1, for example, by operating a predetermined application on the terminal device 600 while accessing the information processing apparatus 100 via the terminal device 600 or receiving information transmitted from the information processing apparatus 100. The predetermined application may be, for example, a dedicated application that operates using information transmitted from the information processing apparatus 100, a web browser that displays a web application provided by the information processing apparatus 100 such that the application can be used, or the like.

In this embodiment, the information processing system 1 is typically used as follows. That is to say, the user measures his or her own biological signals while wearing the measuring device 700 over a predetermined period of time. The time-series information obtained from the measurement is made to be transferred to the terminal device 600. The user then transmits the time-series information stored in the terminal storage unit 610 from the terminal device 600 to the information processing apparatus 100. Accordingly, the information processing apparatus 100 acquires the prediction information and outputs the future information. The terminal device 600 receives the output future information and causes the terminal output unit 660 to display the information on the display device based on the future information. This allows the user to check his or her own future information and information based thereon.

In the case in which such operations of the information processing system 1 are performed, the information processing apparatus 100 performs various operations as follows, for example. These operations are performed by the processing unit 140 executing control operations and the like while using the units.

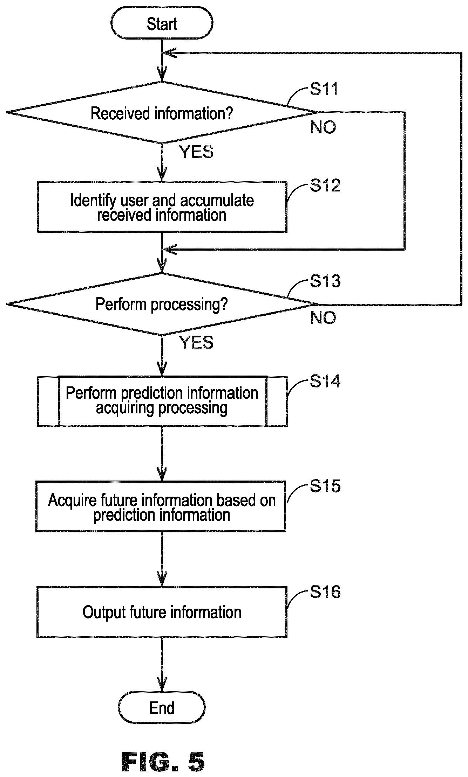

FIG. 5 is a flowchart showing an example of an operation of the information processing apparatus 100.

(Step S11) The processing unit 140 determines whether or not the receiving unit 120 has received information transmitted from the terminal device 600 or the like. If it is determined that it has received the information, the procedure advances to step S12, or otherwise the procedure advances to step S13.

(Step S12) The processing unit 140 identifies the user based on the received information, and accumulates the received information in the user information storage unit 115 in association with the user identifier. For example, if time-series information is transmitted from the terminal device 600 in association with a user identifier, the processing unit 140 accumulates the received time-series information in the user information storage unit 115 in association with that user identifier.

(Step S13) The processing unit 140 determines whether or not a trigger to perform processing such as acquiring prediction information or outputting future information has occurred. In other words, the processing unit 140 determines whether or not a condition for starting processing such as acquiring prediction information or outputting future information has been satisfied. If it is determined that such a trigger has occurred, the procedure advances to step S14. Otherwise, the procedure returns to step S11.

In this case, for example, the above-mentioned trigger can be the provision of an instruction from the user through the terminal device 600 to perform processing such as acquiring prediction information or outputting future information. For example, the above-mentioned trigger can be the transmission of predetermined information corresponding to such an instruction through the terminal device 600, or the like. The trigger is not limited to these. For example, the above-mentioned trigger can be the arrival of a predetermined time, and in this case, the future information can be output periodically. For example, the trigger can be the fulfillment of various conditions such as the new receipt of time-series information or other information.

(Step S14) The processing unit 140 causes the prediction information acquiring unit 145 to perform prediction information acquiring processing. The prediction information acquiring processing will be described later. The prediction information on the user is acquired and accumulated in the user information storage unit 115 through the prediction information acquiring processing.

(Step S15) The processing unit 140 causes the future information output unit 147 to acquire future information based on the prediction information.

(Step S16) The processing unit 140 causes the future information output unit 147 to output the future information. In this embodiment, the processing unit 140 outputs the acquired future information to the target user. That is to say, the processing unit 140 causes the transmitting unit 170 to output the future information to the terminal device 600 that is used by the target user. This allows the terminal device 600 that has received the future information to display the future status of the user.

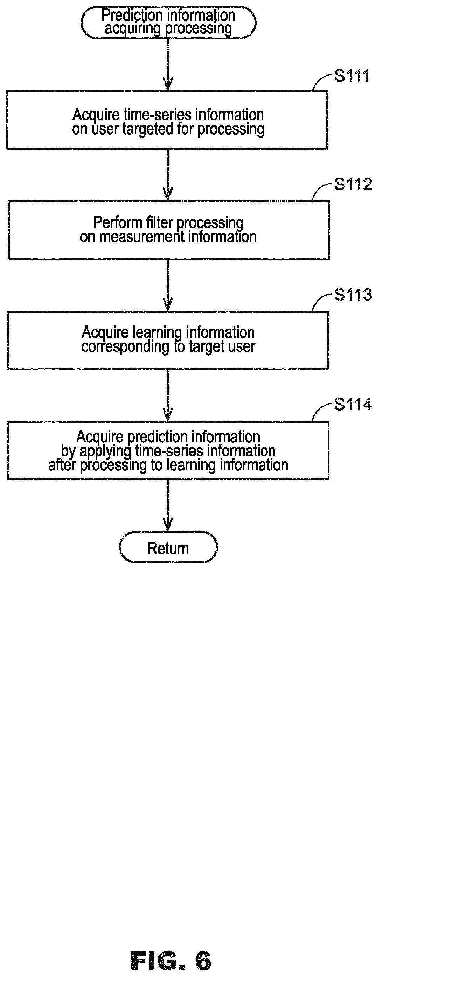

FIG. 6 is a flowchart showing an example of the prediction information acquiring processing of the information processing apparatus 100.

(Step S111) The prediction information acquiring unit 145 acquires time-series information on the target user, from the user information storage unit 115, based on the user identifier of the user.

(Step S112) The prediction information acquiring unit 145 causes the pre-processing unit 146 to perform filter processing on the measurement information.

(Step S113) The prediction information acquiring unit 145 acquires learning information corresponding to the user identifier of the target user, from the learning information storage unit 111.

(Step S114) The prediction information acquiring unit 145 applies the time-series information after the processing by the pre-processing unit 146 to the learning information, thereby acquiring prediction information. The acquired prediction information is accumulated in the storage unit 110 in association with the user identifier of the target user. Thereafter, the procedure returns to the processing in FIG. 4.

Next, specific examples of the acquisition of prediction information by the information processing apparatus 100 according to this embodiment will be described.

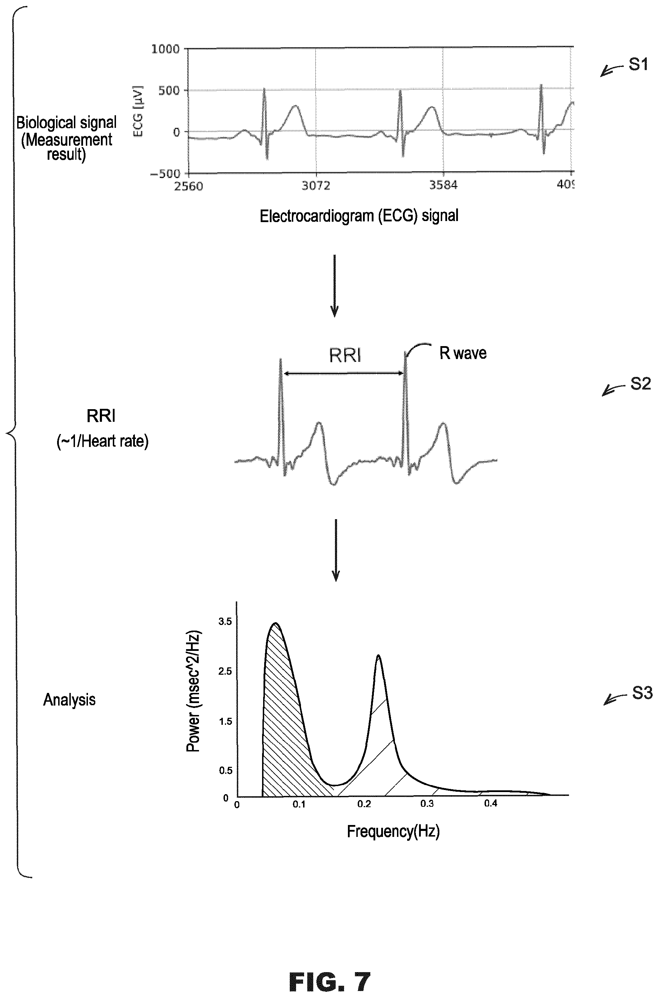

FIG. 7 is a diagram illustrating time-series information in a specific example of this embodiment.

In this specific example, the time-series information is information acquired based on the information showing the ECG of a user as measurement results. The time-series information is information indicating the transition of LF and HF related to heartbeat variability of a user. The time-series information may be information on the transition of LF/HF (ratio of LF to HF). As the prediction information, for example, prediction of the transition of LF or HF over a predetermined period of time in the future may be obtained, or prediction of the transition of LF/HF may be obtained.

LF and HF are values obtained by integrating the power spectrum obtained by frequency analysis of the transition of heartbeat intervals in the respective frequency domain bands (low-frequency band and high-frequency band). LF can be used as an indicator of the degree of activation of the user's sympathetic nervous system, and HF can be used as an indicator of the degree of activation of the user's parasympathetic nervous system. That is to say, LF, HF, and LF/HF can be used as information on the degree of activation of the user's autonomic nervous system.

As shown in the drawing, if the electrocardiogram signal for a predetermined measurement period (e.g., 5 minutes) is obtained as a measurement result (S1), the RRIs (heartbeat intervals: intervals between R waves) is obtained therefrom (S2). LF and HF can be obtained based on the power spectrum obtained by frequency analysis of the transition of RRIs over the predetermined measurement period (S3). LF and HF obtained for each predetermined measurement period can be summarized as time-series information.

Such acquisition of time-series information from the ECG may be performed, for example, by the terminal device 600, but it may also be performed by the measuring device 700 or by the information processing apparatus 100 that acquired the measurement results.

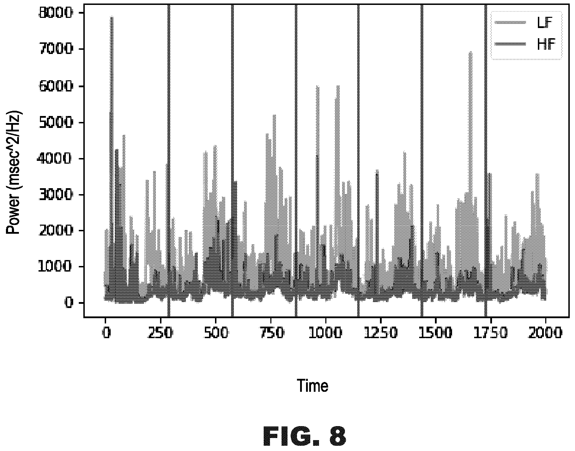

FIG. 8 is a diagram showing an example of time-series information before filter processing in a specific example of this embodiment.

The drawing shows time-series information indicating the transition of LF and HF over time, which is obtained as described above. In this drawing and the next drawing, for example, time-series information for approximately one week is shown. If such time-series information is filtered by the pre-processing unit 146, the result is as follows.

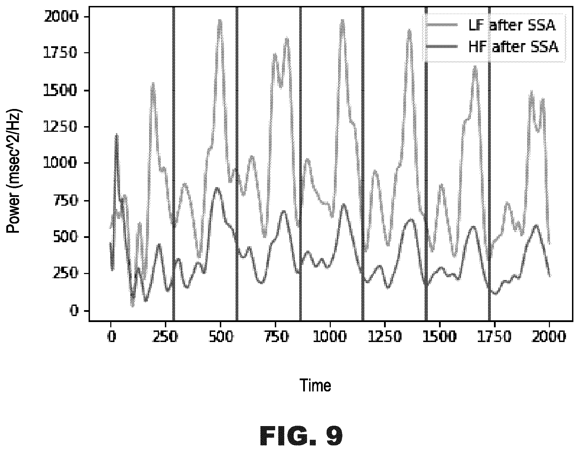

FIG. 9 is a diagram showing an example of time-series information after filter processing in a specific example of this embodiment.

The drawing shows time-series information after the processing obtained by restoring the time-series information, using the results having degrees up to a highest degree of 10 obtained through singular spectrum analysis, in contrast to the previous drawing. Such time-series information after the processing is information that well represents the transition of LF and HF of the user, with the principal components of the time-series information well extracted. By using such time-series information after the processing, it is possible to acquire accurate prediction information.

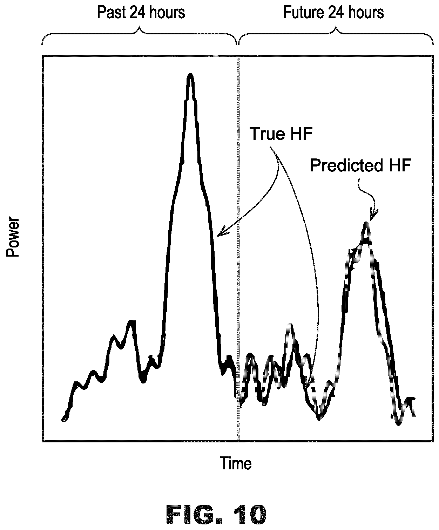

FIG. 10 is a diagram showing an example of prediction information acquired in a specific example of this embodiment.

The drawing shows prediction information on HF (predicted HF) for a predetermined period of time (e.g., 24 hours) in the future obtained from the time-series information on HF (true HF) for a predetermined period of time (e.g., 24 hours) in the past. The time-series information based on the actual subsequent measurement results is also shown for the predetermined time in the future. In this manner, it is possible to acquire prediction information, which is future time-series information, from past time-series information. The prediction information is an accurate prediction of the transition of actual time-series information.

In the thus obtained prediction information, the time period during which HF is dominant over LF by a predetermined degree in a predetermined period of time in the future may be output as future information indicating a time period during which the target user is likely to feel stressed (a time period during which the user is unlikely to be relaxed). The time period during which HF is dominant over LF by a predetermined degree in a predetermined period of time in the future may be output as future information indicating a time period during which the target user is likely to be relaxed (a time period during which the user is unlikely to feel stressed).

As another specific example, instead of using the information constituting the above-described electrocardiogram as measurement results, the time-series information obtained from the pulse waves as the measurement results may be used to obtain prediction information on the user. For example, it is also possible to acquire time-series information indicating the transition of LF and HF and the like from the transition of PPIs (pulse-to-pulse intervals), and acquire prediction information on the transition of LF and HF and the like in the same manner as described above.

Furthermore, it is also possible to acquire information showing the PPG waveform of the user based on the pulse waves of the user as the time-series information, and obtain the future transition of heart rate (HR) and the like of the user based on the time-series information. For example, in the case of obtaining the transition of heart rate, for example, data for learning containing a PPG waveform and an ECG waveform that is paired therewith is prepared, and information indicating the transition of heart rate obtained from the ECG waveform is used so as to be paired with the corresponding PPG waveform, to configure learning information of machine learning. The transition of heart rate can be obtained by applying the time-series information based on measurement results to such learning information. As such learning information, information is used that can constitute a neural network model in which the structure employed in Transformer is used as the encoder structure. This allows the PPG waveform, which can be obtained relatively easily, to be used to acquire prediction information on heart rate with accuracy similar to that in the case in which the information is obtained from ECG. The learning information may be configured such that the instantaneous value of the heart rate can be obtained. The prediction information may be acquired by obtaining the transition of values of indicators other than the heart rate. It is possible to output, for example, future information on the user's peripheral nerve activity, from such prediction information.

Furthermore, it is also possible to obtain prediction information on future brain waves of the user, by acquiring information showing brain waves as time-series information. The thus obtained prediction information may be used to output future information on the activation of the user's central nervous system. For example, information useful for the user can be obtained by appropriately predicting the degree of brain arousal during sleep and the degree of brain arousal during waking.

As explained above, the information processing apparatus 100 can acquire prediction information on future time-series information for an individual user, from time-series information on a status of a living body. Accordingly, it is possible to acquire information on the future status of the user. The user can provide the time-series information to the information processing apparatus 100, for example, by measuring biological signals in a simple method using the measuring device 700 that is a wearable device. Accordingly, the user can easily obtain information on his or her future state. In this embodiment, prediction information is acquired using learning information that constitutes a neural network model using Transformer. Accordingly, it is easy to acquire accurate prediction information.

The storage unit 110 and the terminal storage unit 610 described above are preferably non-volatile recording media, but they may alternately be realized by volatile recording media. Information and the like acquired by each apparatus are stored in the corresponding units, but the procedure in which information and the like are stored is not limited to this. For example, information and the like may be stored via a recording medium, information and the like transmitted via a communication line or the like may be stored, or information and the like input via an input device may be stored.

Furthermore, the processing unit 140 and the terminal processing unit 640 described above may be typically realized by MPUs, memories, or the like. Typically, the processing procedure of the processing unit 140 and the terminal processing unit 640 is realized by software, and the software is stored in a recording medium such as a ROM. Note that the processing procedure may be realized by hardware (dedicated circuits).

Furthermore, the information that can be accepted by the accepting unit 130 or the terminal accepting unit 630 may be input by any input part such as a numeric keypad, a keyboard, a mouse, or a menu screen. The accepting unit 130 and the terminal accepting unit 630 may be realized by device drivers for an input part such as a numeric keypad or a keyboard, control software for a menu screen, or the like.

Furthermore, the receiving unit 120 and the terminal receiving unit 620 are typically realized by wireless or wired communication parts, but they may also be realized by broadcast receiving parts.

Furthermore, the transmitting unit 170 and the terminal transmitting unit 670 are typically realized by wireless or wired communication parts, but they may also be realized by broadcasting parts.

The information processing apparatus 100 may be constituted by a single server, multiple servers that operate in cooperation with each other, or a computer or other device built into other equipment. It will be appreciated that the servers may be so-called cloud servers, ASP servers, or the like, and there is no limitation on the type thereof.

The processing in this embodiment may be realized by software. The software may be distributed by software downloads or the like. Furthermore, the software may be distributed in a form where the software is stored in a recording medium such as an optical disk. The software that realizes the information processing apparatus 100 in this embodiment is the following sort of program. Specifically, this program is a program that is executed on a computer of the information processing apparatus 100, the program causing the computer of the information processing apparatus 100 to function as: a biological information acquiring unit that acquires time-series information on a status of a living body based on measurement results of a biological signal of a user; a prediction information acquiring unit that acquires prediction information on future time-series information on the user using a machine learning method, using learning information configured using time-series information acquired from measurement results of a biological signal in the past, and the time-series information; and a future information output unit that outputs future information on a future status of the user based on the prediction information.

### Embodiment 2

In Embodiment 2, an information processing system 2 uses an information processing apparatus that can acquire score information containing a score indicating a present or future physical condition of an individual user. The score information is acquired using measurement information on a status of a living body based on measurement results of a bioelectric potential of a user. In this embodiment, output information on the score is output based on the acquired score information. This allows the user to learn about his or her own present or future physical condition. Hereinafter, the thus configured information processing system 2 will be described.

The score indicating a present or future physical condition of a user is, for example, a value that represents the degree of the user's physical condition being good, in a score format. For example, a higher score indicates that the user is in better physical condition. It is also possible that a lower score indicates that the user is in better physical condition. The score may be a value expressed as a rank, or a statement or the like indicating the physical condition. The degree of the physical condition being good is, for example, the degree of stress being light. That is to say, the score may indicate the user's stress level (the degree at which the user feels stressed). The physical condition may be the degree of wakefulness, the degree of feeling fatigue, the body temperature, or the like, or it may be expressed as a combination of two or more of these elements.

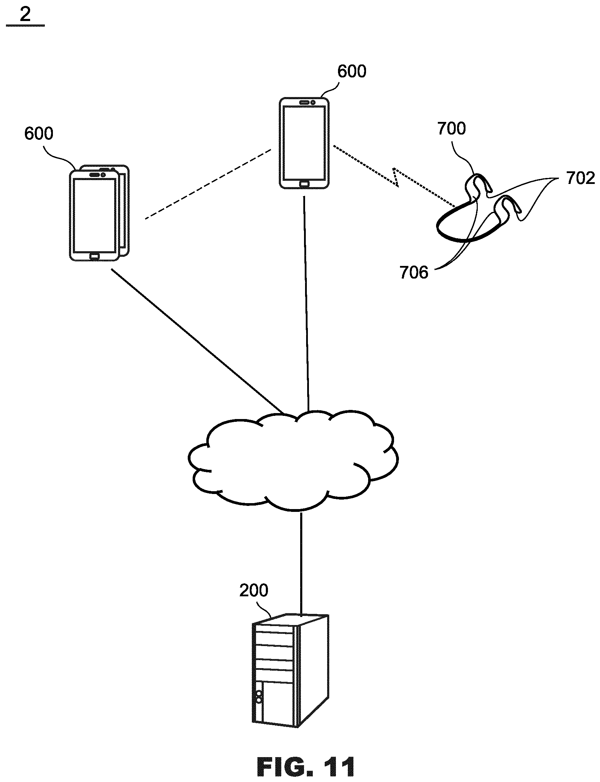

FIG. 11 is a diagram showing the outline of the information processing system 2 according to Embodiment 2 of the present invention.

In this embodiment, the information processing system 2 includes an information processing apparatus 200, the above-described terminal devices 600, and the above-described measuring devices 700. That is to say, the information processing system 2 is different from the information processing system 1 according to Embodiment 1, in that the information processing apparatus 200 is included instead of the above-described information processing apparatus 100. The information processing apparatus 200 and the terminal devices 600 can communicate with each other via a network. The configuration of the information processing system 2 is not limited to this. There is no limitation on the number of apparatuses of each type included in the information processing system 2, and other types of apparatuses may also be included in the information processing system 2.

In Embodiment 2, each measuring device 700 that is a headset includes measuring device output units 706. The measuring device output units 706 are, for example, speakers positioned near the user's ears when the measuring device 700 is worn by the user. The measuring device output units 706 are, for example, sound output units that output a user-hearable sound by vibrating air or an object. The measuring device 700 is configured to be able to output a sound from the measuring device output units 706. The user can hear the sound output from the measuring device 700 by using the measuring device 700. The measuring device output units 706 may include a display screen or the like that can be viewed by the user, instead of or in addition to the speakers. In this case, there is no limitation on the form of the display screen.

In Embodiment 2 as well, the measurement information contains information obtained by measuring a value related to a human bioelectric potential. This information may be said to be information obtained by measuring a value related to a human bioelectric potential. In this embodiment as well, measurement information that is time-series information is used. That is to say, the time-series information described in Embodiment 1 above may be included in the measurement information in Embodiment 2. The measurement information in Embodiment 2 is not limited to this. That is to say, it is sufficient that the information processing apparatus 200 is configured to use measurement information on a bioelectric potential at two or more points in time measured by the measuring device 700. In other words, the measurement information is not necessarily limited to what is referred to as time-series information, and in this embodiment, information obtained by measuring a user in a time series is used as the measurement information.

The users of the information processing system 2 can use the information processing system 2 by using the terminal devices 600 and the measuring devices 700. In the drawing, for example, portable information terminal devices such as so-called smartphones are shown as the terminal devices 600, but the configuration of the terminal devices 600 and the measuring devices 700 can be changed in various manner, as in Embodiment 1.

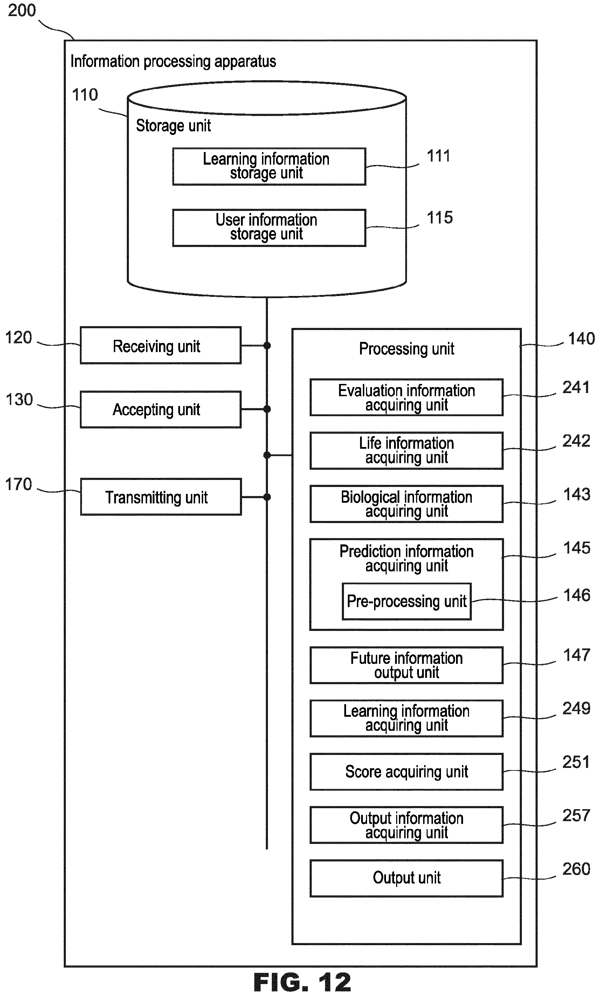

FIG. 12 is a block diagram of the information processing apparatus 200.

The information processing apparatus 200 is, for example, a server apparatus. The information processing apparatus 200 includes the storage unit 110, the receiving unit 120, the accepting unit 130, the processing unit 140, and the transmitting unit 170. Hereinafter, the configuration of the information processing apparatus 200 will be described mainly focusing on differences from the configuration of the information processing apparatus 100 according to Embodiment 1.

In Embodiment 2, learning information for score acquisition acquired in advance is stored in the learning information storage unit 111, in addition to the learning information similar to that in Embodiment 1. In this embodiment, the learning information for score acquisition is generated such that input information containing measurement information obtained by measuring two or more users in the past is taken as information that is input and information on a score as will be described later is taken as information that is output. The learning information is generated using a so-called machine learning method. The learning information is, for example, generated by a learning information acquiring unit 249 and stored in the learning information storage unit 111 as described later, but there is no limitation to this. That is to say, learning information generated in an apparatus different from the information processing apparatus 200 may be stored in the learning information storage unit 111.

Furthermore, in Embodiment 2, user information stored in the user information storage unit 115 may contain life information on a user. The life information is, for example, transmitted from the terminal device 600 that is used by the user, received by the receiving unit 120, and stored in the user information storage unit 115.

The life information is information on the user's life status. The life status includes various elements related to the user's behavior, state, and the like. In the following embodiment, the life information contains, for example, eating-and-drinking information and activity status information. That is to say, the life status includes, for example, the user's eating-and-drinking status, the user's activity status, and the like. However, there is no limitation to these, and the life information may contain information on other elements, or information on only some of these elements.

The eating-and-drinking information is information on the user's eating-and-drinking status. The eating-and-drinking information may contain information from various viewpoints such as the amount of water consumed, the amount of alcohol consumed, and the content of a meal. The eating-and-drinking information may be conceptually divided into food information on food and eating habits and drink information on drink consumption. More specifically, for example, in this embodiment, the eating-and-drinking information includes, but is not limited to, at least one of information on the amount of water consumed, information on whether or not alcohol was consumed or the amount of alcohol consumed, information on whether or not a meal was taken or the content of a meal, and information on whether or not a particular group of food was consumed or the amount thereof consumed. The particular group of food refers to food that belongs to a particular group among the groups classified as meat, dairy products, vegetables, and the like, for example, but the viewpoint and granularity of such "group" classification are not limited to this. One or both of the food information and the drink information may include those related to so-called supplements (health foods). The eating-and-drinking information is, but is not limited to, information that is input by an evaluator such as a user who performed evaluation of the eating-and-drinking status. For example, the eating-and-drinking information may be information resulting from measurement or evaluation by an evaluation device or the like. For example, the eating-and-drinking information may contain a time period during which the user ate and drank and a measured value related to mastication, which are ascertained based on the results of myoelectric measurements performed by the measuring device 700.

The activity status information is information on the user's activity status. The activity status herein refers to a status related to the user's state or lifestyle habits (i.e., actions and behaviors that the user repeatedly performs in daily life) excluding the eating-and-drinking status. That is to say, the activity status information may include information on lifestyle habits excluding the eating-and-drinking information. In this embodiment, the activity status information includes, but is not limited to, at least one of basic information, sleep information on sleep (sleep duration, sleep quality, etc.), and exercise information. The basic information is information on the user's type and characteristics. For example, the basic information may include various types of information such as the user's height, weight, age, sex, body fat percentage, muscle mass, lifestyle habits (e.g., whether or not the user smokes, whether or not the user consumes alcohol, etc.), and the like. The exercise information is information on the user's exercise or status. For example, the exercise information may include information on measurement results of pulse (pulse rate), respiratory rate, body temperature, blood pressure, and blood oxygen level. The exercise information may include information on acceleration obtained by measuring the user. The acceleration may include, for example, values measured at the head, wrist, and other predetermined sites, as well as values measured while the user is holding the measuring device. The information on acceleration may be treated as the number of steps taken, the caloric expenditure, the intensity of exercise, the frequency of exercise, or the like. The activity status information may be, for example, information that is acquired by the terminal device 600 that the user is holding or wearing or by an activity meter (not shown) that is included in the measuring device 700, or it may be information that is input by an evaluator such as the user as a result of performing evaluation. The activity status information may include user's responses to questionnaires, information generated based on those responses, information on the user's medical history, and the like, or it may be information acquired from an external database or the like in which user information is recorded.

The processing unit 140 includes, for example, the biological information acquiring unit 143, the prediction information acquiring unit 145, and the future information output unit 147, as in Embodiment 1. In this embodiment, the biological information acquiring unit 143 acquires past time-series information based on actual measurement results, as the measurement information. The processing similar to that in Embodiment 1 is performed based on this information, and thus prediction information, which is future time-series information, is acquired and output by the future information output unit 147. That is to say, prediction information is acquired based on input information, using learning information configured such that input information on measurement information, which is past time-series information, is taken as input and future measurement information is taken as output. In Embodiment 2, the output of future information is, for example, a hand-off of future measurement information, which is prediction results, to the subsequent processing in the processing unit 140, or accumulating the information in the storage unit 110.

Furthermore, the processing unit 140 includes an evaluation information acquiring unit 241, a life information acquiring unit 242, a learning information acquiring unit 249, a score acquiring unit 251, an output information acquiring unit 257, and an output unit 260.

The evaluation information acquiring unit 241 acquires evaluation information corresponding to the score information. The score information is information that is acquired by the score acquiring unit 251 as will be described later. The evaluation information may be said to be, for example, information indicating the degree of conformity between a score based on score information (i.e., a predicted score) and an actual physical condition of a user (hereafter simply referred to as a "physical condition"). The evaluation information may be a binary value indicating whether or not the score is indicative of the physical condition, or it may be a value indicating the degree to which the score corresponds to the physical condition. It may also be the score itself that indicates the physical condition. The evaluation information acquiring unit 241 may acquire, as the evaluation information, information that is input by the user and that indicates a result determined by the user subjectively, or it may acquire evaluation information based on measurement information and the like obtained by the measuring device 700. For example, in the case in which score information indicating a score at some future point in time is acquired, the evaluation information may be acquired based on the results of a comparison between the score calculated based on the measurement information subsequently measured at that point in time and the acquired score. Such evaluation information may be said to be information on the score reflecting the actual physical condition.

The life information acquiring unit 242 acquires life information. In this embodiment, the life information includes, but is not limited to, eating-and-drinking information and activity status information (i.e., exercise information and sleep information). The life information acquiring unit 242 acquires information stored in the user information storage unit 115, for example. In addition, the life information acquiring unit 242 may also acquire basic information on the user.

The learning information acquiring unit 249 generates learning information for score acquisition, using a machine learning method. The machine learning method can be used as follows, for example. That is to say, a learning model in which input information containing at least measurement information is taken as input and information on the score (output value) is taken as output is configured using a machine learning method. For example, two or more pairs of input information and output values are prepared in advance, and the two or more pairs of information are given to a module for configuring a learning model of machine learning to configure a learning model. That is to say, the learning information acquiring unit 249 generates learning information using two or more pieces of training data each having input information containing at least measurement information and a score regarding a physical condition of a user. The learning model may also be said to be a classifier. There is no limitation on the machine learning method, and examples thereof include deep learning such as convolutional neural networks (CNNs), random forests, and SVR. For example, functions in various machine learning frameworks and various existing libraries, such as fastText, tinySVM, random forest, and TensorFlow, can be used for the machine learning.

The information on the score may be, for example, a score itself, or information having a predetermined correspondence relationship with the score (e.g., a variable in a predetermined function, information associated with the score in a predetermined table, etc.). The information having a predetermined correspondence relationship with the score may be a value corresponding to the same element (brain waves, myoelectricity, cardiac electrical activity, etc.) as the measurement information. The information on the score may be score information, which will be described later.

The learning information acquiring unit 249 accumulates the configured learning model as learning information in the learning information storage unit 111. Accordingly, the processing unit 140 can acquire generated learning information from the learning information storage unit 111 in the case of using the learning information.

In this case, the combination of input information and an output value that are used to generate learning information for score acquisition may be prepared in advance. In this embodiment, for users whose score information is to be newly acquired, learning information configured using input information containing measurement information obtained by measuring two or more users in the past is used. This makes it possible to acquire scores from the beginning of the measurement even for users whose scores have not been acquired in the past. It is also possible that for each user, a combination of input information and an output value that correspond to the user may be prepared, and in this case, the generated learning information may be accumulated in the learning information storage unit 111 in association with a user identifier of the user. The learning information for score acquisition may be generated such that it can be commonly used for multiple users, and in this case, for example, it may be generated such that it can be commonly used for multiple users who are identical in terms of a predetermined attribute. Such learning information and the like can be configured by various methods, such as by using training data of multiple users together.

Furthermore, in Embodiment 2, the learning information acquiring unit 249 acquires, for a user, evaluation information corresponding to the score information acquired by the score acquiring unit 251, and acquires learning information obtained by performing re-learning using input information used to acquire the score information and evaluation information. In other words, the learning information acquiring unit 249 regenerates, for each user, learning information for score acquisition using a combination of input information containing measurement information newly obtained by the measuring device 700 and information on the score reflecting the actual physical condition corresponding thereto, at a predetermined point in time. This re-learning processing may be performed for each group of two or more users. In the case in which the learning information common to all users is used, the re-learning processing may be performed as appropriate, without distinguishing between users.

The learning information acquiring unit 249 may be configured to acquire learning information stored in another apparatus that is not the information processing apparatus 200, from that apparatus. For example, if the learning information stored in another apparatus is always used, the information processing apparatus 200 may not be provided with the learning information storage unit 111.

The score acquiring unit 251 acquires score information based on the measurement information as follows, for example. The score information is information containing a score indicating a present or future physical condition of a user. The measurement information may be, for example, measurement information acquired by the biological information acquiring unit 143, and measurement information that is future information output by the future information output unit 147. The future information may be said to be information acquired using learning information configured such that input information on past measurement information is taken as input and future measurement information is taken as output.

The future information that is future measurement information may be acquired and generated in the same manner as in Embodiment 1 above, but this processing may be interpreted as being performed by the score acquiring unit 251. That is to say, in Embodiment 2, the score acquiring unit 251 may be interpreted as performing the processing of the prediction information acquiring unit 145 and the future information output unit 147.

The score information is, for example, information in which information on a future time is associated with a score indicating a physical condition at that time, or information indicating the transition of the score. That is to say, the score information may contain information on a score indicating a physical condition at one or multiple specific points in time in the present or future. The score information may contain information indicating the transition of a score indicating a physical condition over a predetermined period of time, and the predetermined period of time may or may not include a future period. The score information may be the value of the score itself.

Specifically, for example, in the case in which measurement information indicating the transition of HF and LF over time is used, the user's stress level can be numerically identified. That is to say, a score indicating the degree of stress felt by the user being light or a score indicating the degree of stress felt by the user being heavy can be numerically identified. However, there is no limitation to this, and for example, in the case in which measurement information that is brain waves is used, the user's level of sleep-wakefulness (an example of physical condition) can be numerically identified as scores. For example, if the user is awake and feels sleepy, the score will be lower than if the user is not. Conversely, if the user is able to maintain a high level of concentration, the score will be higher than if the user is not. For example, in the case in which information on the heart rate is used as measurement information, the physical fitness and physical fatigue (an example of physical condition) can be numerically identified as scores based on this information. For example, if the heart rate is high relative to the user's activity level, the score will be lower than if it is not. In addition, various other states may be numerically identified as scores based on measurement information on brain waves, pulse waves, myoelectricity, and the like.

In Embodiment 2, the score acquiring unit 251 acquires a measurement score based on measurement information and a life score based on life information, respectively. Then, score information indicating a physical condition is acquired based on the measurement score and the life score.

The score acquiring unit 251 acquires a measurement score based on input information containing at least measurement information at multiple points in time. The score acquiring unit 251 acquires a measurement score, for example, by applying the input information to the learning information stored in the learning information storage unit 111. The measurement score is a value obtained based on the measurement information. In Embodiment 2, the score acquiring unit 251 may acquire future measurement information, for example, by applying learning information configured such that future measurement information is taken as output to input information containing measurement information at multiple present or past points in time. The acquired measurement information may be used to acquire a future measurement score. The score acquiring unit 251 accumulates the acquired measurement score in the user information storage unit 115 in association with the user identifier.

The learning information does not necessarily have to be used to acquire the measurement score or the score information. For example, the score acquiring unit 251 may be configured to obtain measurement information at a predetermined point in time using a statistical method such as linear regression based on the measurement information at two or more points in time, and to acquire a measurement score and the like corresponding to the measurement information using a predetermined function or table, for example.

Furthermore, the score acquiring unit 251 may compare the content of the measurement information (e.g., the transition of a biological signal over a predetermined period of time) and a predetermined criterion, and calculate a measurement score, for example, by using a predetermined calculation formula according to the comparison results. Also, multiple threshold values may be prepared as criteria, and predetermined points corresponding to the conditional range that encompasses the content of the measurement information may be reflected in the measurement score. The points to be reflected may be specified in advance based on information mapped in a space composed of multiple viewpoint axes (e.g., an n-dimensional look-up table), and the specified points may be reflected in the measurement score.

Furthermore, the score acquiring unit 251 acquires a life score based on life information acquired by the life information acquiring unit 242. The score acquiring unit 251 accumulates the acquired life score in the user information storage unit 115 in association with the user identifier.

The life score is a numerical value, code indicating a grade, or the like indicating the degree of the state of a subject matter such as the user's lifestyle habits, status, or eating-and-drinking behavior being good. The score acquiring unit 251 acquires a life score based on the life information, for example, based on whether or not the contents of the various types of life information satisfy predetermined conditions. The predetermined conditions can be set for each element included in the life information for each viewpoint. It is possible to acquire a life score, for example, by reflecting a first predetermined score in the life score if a predetermined condition is met, and reflecting a second predetermined score in the life score if not. The score acquiring unit 251 may acquire a score for each element included in the life information individually and use each acquired score to acquire a living score. For example, a value derived by a predetermined function including variables for respective scores, such as an added value of the scores or an average value of the scores, may be acquired as a life score. Also, multiple threshold values may be prepared as criteria, and predetermined points corresponding to the conditional range that encompasses the content of the life information may be reflected in the life score. The points to be reflected may be specified in advance based on information mapped in a space composed of multiple viewpoint axes (e.g., an n-dimensional look-up table), and the specified points may be reflected in the life score.

For example, in the case in which a measured value of blood pressure is used as the life information, if the measured value of blood pressure for the most recent predetermined period of time is greater than a predetermined value, the life score will be calculated to be lower than if it is not. For example, in the case in which the amount of calories taken in is used as the life information, if the amount of calories taken in in the past few days is greater than a predetermined value, the life score will be calculated to be lower than if it is not. For example, in the case in which the amount of calories consumed is used as the life information, if the amount of calories consumed through exercise in the past few days is within a predetermined range, the life score will be calculated to be higher. The calculation of the life score is not limited to this, and may be set according to the type and the like of life information actually used.

The score acquiring unit 251 acquires the score information using the acquired measurement score and life score. The score acquiring unit 251 acquires the score information, for example, in which a value derived by a predetermined function including variables for respective scores, such as an added value of the scores or an average value of the scores, is taken as a score indicating a physical condition. The score acquiring unit 251 accumulates the acquired score information in the user information storage unit 115 in association with the user identifier. The score acquiring unit 251 may acquire the score information, using learning information configured using a machine learning method. The learning information may be, for example, information configured such that the measurement score and the life score are taken as input and the score indicating a physical condition is taken as output. As necessary, the measurement score and the life score may be, for example, normalized and used to acquire the score information.

In Embodiment 2, the life information may not be used. The score acquiring unit 251 may acquire the score information using only the measurement information. The score acquiring unit 251 may be configured to acquire information on the score using input information containing the measurement information and the life information, and learning information, and to acquire the score information based on the information.

For example, the input information may contain the measurement information as well as information obtained by measuring at least one of the user's blood pressure, pulse, blood oxygen level, body temperature, respiratory rate, and acceleration. In this case, the score acquired may provide a more accurate indication of the user's physical condition.

Furthermore, for example, the input information may contain the measurement information as well as eating-and-drinking information on the user's eating-and-drinking behavior. In this case, the score acquired may provide a more accurate indication of the user's physical condition.

The score acquiring unit 251 may acquire the score, based on the measurement score or life score acquired in the past for the user concerned stored in the user information storage unit 115, input information or life information at a past point in time, or the like. For example, the present score may be acquired by reflecting the present points in the previously acquired score. The score may be acquired using the input information or life information over a predetermined period of time in the past and the input information or life information in the present, for example, using an average of these values.

Factors such as the conditions set with respect to acquiring such scores, the reference values to be compared, the scores serving as the criteria, the points to be reflected in the scores, the method for reflecting points in the scores (addition, subtraction, multiplication, etc.), and the like may be set in a timely manner or the learning information to be used may be selected. This processing can be performed based on the user's life information, more specifically, basic information and the like, for example. That is to say, the score acquiring unit 251 may acquire the score based on the user's basic information. Specifically, for example, the score acquiring unit 251 may be configured to apply different factors and learning information based on the user's sex and age to acquire a score.

The output information acquiring unit 257 acquires output information on the score based on the score information. The output information on the score is, for example, the following information. In Embodiment 2, the output information is information that is used in the terminal device 600 to inform the user of the score acquired. For example, the output information may be information having text or code indicating the score.

Furthermore, for example, the output information may be information for outputting information such as score values by means of images or sound. For example, the output information acquiring unit 257 may acquire output information for outputting a sound from the measuring device 700. Various types of information may be used as such output information, including, for example, sound data and data to make the sound source installed in the device play in a predetermined manner. For example, if information such as score values is output as a sound from the measuring device 700, the user can easily acquire information on his or her own physical condition without using his or her sense of sight.

In this case, the output information acquiring unit 257 may acquire a largest value of a score of score information that may be acquired for the user, and acquire output information corresponding to a relationship between the largest value and the score information acquired by the score acquiring unit 251. The largest value may be said to be, for example, the score at the best physical condition. For example, the largest value may be, but is not limited to, the score identified based on the measurement information measured when it is determined that the user is in the best physical condition. A predetermined largest value may be acquired uniformly for all users. The output information acquiring unit 257 can express, for example, the score indicating the present physical condition as a percentage with the best physical condition being 100 percent, as corresponding to a relationship between the largest value and the score information. In this manner, the score can be shown as a value relative to the largest value, allowing the user to intuitively understand the present physical condition. The largest value may be, for example, the score in the state of the poorest physical condition.

In Embodiment 2, the output information acquiring unit 257 acquires advice on eating-and-drinking behavior of a user to ensure that a score higher than the score of the score information acquired by the score acquiring unit 251 is obtained in the future. The output information acquiring unit 257 acquires the acquired advice as output information together with the information on the score. If such output information is output, the user can act to be in better physical condition in the future with reference to the content of the advice.

The output information acquiring unit 257 can acquire various forms of advice as follows, for example. For example, scores at two or more points in time from the past to the future are compared, and the results of the comparison, that is, the transition pattern, the amount of change, and the like in the physical condition are specified. Then, advice corresponding to the specified pattern, amount of change, or the like is extracted from a table prepared in advance and acquired as advice to be included in the output information.

Such advice may be prepared as appropriate depending on the type of measurement information or the life information used to output the score or the like. For example, such advice may include the following in the case in which the score represents the stress level. For example, advice on activities to make the user feel less stressed, such as "You may become more stressed in the future. Exercise" or "Tomorrow may be a stressful day for you. Why don't you take a longer rest?", may be acquired. For example, advice on eating-and-drinking, such as "Stress is likely to increase in an hour. Why don't you take a rest over a cup of herbal tea?", may be acquired. Also, advice useful for reconsidering the user's action schedule, such as "The score will peak in two hours. Let's adjust your schedule", may be acquired. The advice may also include information on the score that is expected to be acquired if the user performs a predetermined action according to the content of the advice. For example, advice such as "Drinking a cup of coffee now will help you maintain concentration for about 2 hours with moderate stress" may be acquired. In the case in which the score represents the sleep-waking level, for example, advice useful for reconsidering the user's action schedule, such as "The score will peak in two hours. Let's adjust your schedule", may be acquired. For example, the advice may also include information on the score that is expected to be acquired if the user performs a predetermined action according to the content of the advice, such as "If you drink 200 ml of green tea now, you can maintain a high arousal level for another two hours".

The output unit 260 outputs the output information acquired by the output information acquiring unit 257. In this embodiment, the output unit 260 performs output, for example, by transmitting the output information to the terminal device 600, but there is no limitation to this. The output may be performed by any of displaying the information on a display screen or the like, printing the information on paper or the like, and transmitting the information to other devices. For example, the output may be performed by displaying the output information based on the score in the form of text or images on a display screen provided by the information processing apparatus 200, based on the score information. The output unit 260 may be considered to include or to not include an output device such as a display screen or a speaker. The output unit 260 may be realized by driver software for an output device, a combination of driver software for an output device and the output device, or the like.

Next, an example of the operation of the information processing apparatus 200 performed when a user uses the information processing system 2 according to Embodiment 2 will be described. In this embodiment, the user can use the information processing system 2, for example, by operating a predetermined application on the terminal device 600 while accessing the information processing apparatus 200 via the terminal device 600 or receiving information transmitted from the information processing apparatus 200. The predetermined application may be, for example, a dedicated application that operates using information transmitted from the information processing apparatus 200, a web browser that displays a web application provided by the information processing apparatus 200 such that the application can be used, or the like.

In this embodiment, the information processing system 2 is typically used as follows. That is to say, the user measures his or her own biological signals while wearing the measuring device 700. The measurement information obtained from the measurement is made to be transferred to the terminal device 600. The user then transmits the measurement information stored in the terminal storage unit 610 from the terminal device 600 to the information processing apparatus 200. The life information and the like stored in the terminal storage unit 610 may also be transmitted from the terminal device 600 to the information processing apparatus 200.

Accordingly, the information processing apparatus 200 acquires the score information and outputs the output information. The terminal device 600 receives the output information that has been output and causes the terminal output unit 660 to display the score on the display device. This allows the user to check information on his or her own present or future physical condition. Since the physical condition is indicated as the score, the user can easily check information on his or her own physical condition. In this case, if the advice to the user acquired by the output information acquiring unit 257 is displayed together with the score, the user can raise his or her own awareness and take actions to improve his or her physical condition by referring to the advice.

In the case in which such operations of the information processing system 2 are performed, the information processing apparatus 200 performs various operations as follows, for example. These operations are performed by the processing unit 140 executing control operations and the like while using the units.

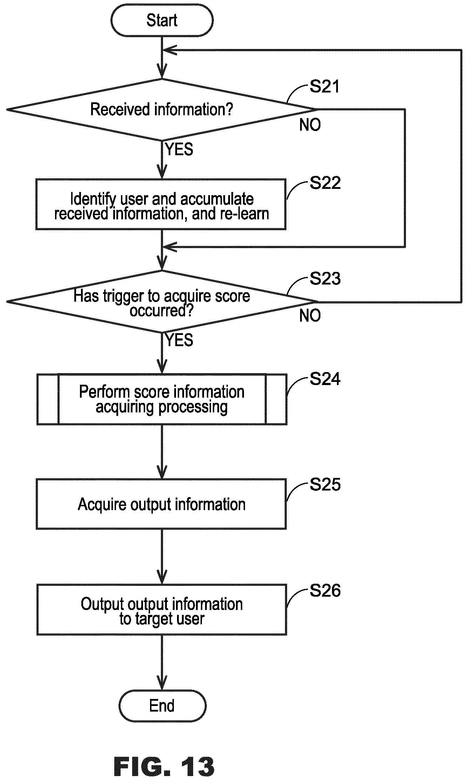

FIG. 13 is a flowchart showing an example of an operation of the information processing apparatus 200.

(Step S21) The processing unit 140 determines whether or not the receiving unit 120 has received information transmitted from the terminal device 600 or the like. If it is determined that it has received the information, the procedure advances to step S22, or otherwise the procedure advances to step S23.

(Step S22) The processing unit 140 identifies the user based on the received information, and accumulates the received information in the user information storage unit 115 in association with the user identifier. For example, if life information, measurement information, or the like is transmitted from the terminal device 600 in association with a user identifier, the processing unit 140 accumulates the received information in the user information storage unit 115 in association with the user identifier.

In this case, the processing unit 140 outputs future information on future measurement information based on the received measurement information, as in Embodiment 1. Accordingly, the information received from the terminal device 600 and the measurement information including the future information are accumulated in the user information storage unit 115. It is also possible to use only the measurement information received from the terminal device 600 to acquire score information, without performing such processing.

In this case, for example, if the previous output information is output and evaluation information corresponding thereto is transmitted from the terminal device 600, the evaluation information may be used to perform re-learning of the learning information. This enables a more accurate score to be acquired.

(Step S23) The processing unit 140 determines whether or not a trigger to acquire a score indicating a physical condition has occurred. In other words, the processing unit 140 determines whether or not a condition for starting the acquisition of a score has been satisfied. If it is determined that such a trigger has occurred, the procedure advances to step S24. Otherwise, the procedure returns to step S21.

Examples of the above-mentioned trigger can include, but are not limited to, the provision of an instruction from the user through the terminal device 600 to acquire a score (transmission of predetermined information corresponding to the instruction) and the like. For example, the trigger can be the fulfillment of various conditions such as the arrival of a predetermined time, the new receipt of measurement information, or the like.

(Step S24) The processing unit 140 causes the score acquiring unit 251 to perform score information acquiring processing. The score information acquiring processing will be described later. The score information is acquired and accumulated in the user information storage unit 115 through the score information acquiring processing.

(Step S25) The processing unit 140 causes the output information acquiring unit 257 to acquire output information, using the acquired score information and the like.

(Step S26) The processing unit 140 causes the output unit 260 to output the acquired output information to the target user. That is to say, the processing unit 140 causes the transmitting unit 170 to output the output information to the terminal device 600 that is used by the user targeted for score calculation. This allows the terminal device 600 that has received the information to perform display or the like regarding the score.

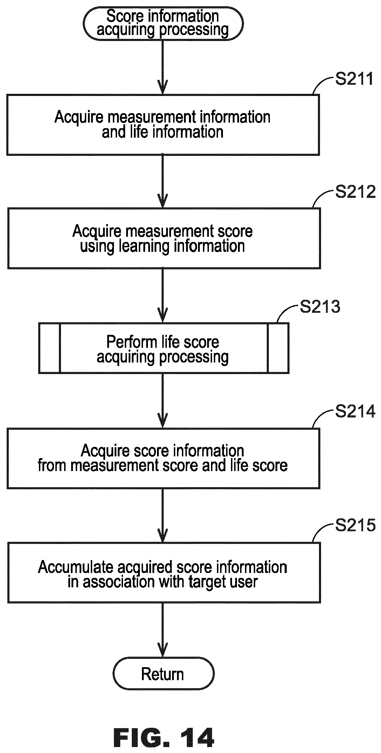

FIG. 14 is a flowchart showing an example of score information acquiring processing the information processing apparatus 200.

(Step S211) The score acquiring unit 251 acquires measurement information and life information or the like regarding a target user, from the user information storage unit 115, based on the user identifier of the user.

(Step S212) The score acquiring unit 251 performs processing that acquires a measurement score, using the measurement information and the learning information.

(Step S213) The score acquiring unit 251 performs processing that acquires a life score, using the life information. The calculation of the life score will be described later.

(Step S214) Once the measurement score and the life score are obtained, the score acquiring unit 251 acquires score information on a score indicating a physical condition. At this time, the score acquiring unit 251 calculates the score, for example, by adding or multiplying in a predetermined manner using the measurement score and the life score, as described above.

(Step S215) The score acquiring unit 251 accumulates the acquired score information and the like in the user information storage unit 115 in association with the user identifier of the target user. Thereafter, the procedure returns to the upper-level processing.

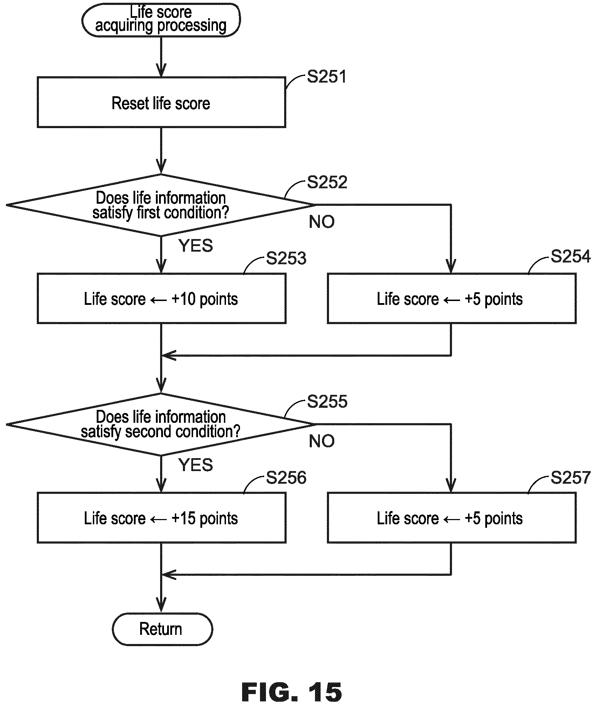

FIG. 15 is a flowchart showing an example of life score acquiring processing of the information processing apparatus 200.

In this case, information on elements that can be included in the life information will be collectively described as life information, without being distinguished from each other. If information on multiple elements is included in the life information, it is sufficient to perform processing such as determining whether or not the following conditions are satisfied for each element. In the following explanation, a first condition and a second condition related to the life information are used, but the determination may be performed for only one condition or for three or more conditions.

(Step S251) The score acquiring unit 251 resets each life score to its initial value (e.g., zero).

(Step S252) The score acquiring unit 251 determines whether or not the life information satisfies a predetermined first condition. If it is determined that the life information satisfies the first condition, the procedure advances to step S253, or otherwise the procedure advances to step S254.

(Step S253) The score acquiring unit 251 adds 10 points to the life score. The procedure advances to step S255.

(Step S254) The score acquiring unit 251 adds 5 points to the life score. The procedure advances to step S255.

(Step S255) The score acquiring unit 251 determines whether or not the life information satisfies a predetermined second condition. If it is determined that the life information satisfies the second condition, the procedure advances to step S256, or otherwise the procedure advances to step S257.

(Step S256) The score acquiring unit 251 adds 15 points to the life score.

(Step S257) The score acquiring unit 251 adds 5 points to the life score.

If step S256 or step S257 is completed, the score acquiring unit 251 acquires a life score as a result of the above, and the procedure returns to the upper-level processing.

If the information processing apparatus 200 performs operations as described above, score information on the user is acquired and output information is output.

Next, specific examples of the acquisition of score information and the output of information to a user by the information processing apparatus 200 according to Embodiment 2 will be described.

An specific example of the output of information to a user is as follows, for example. In the following specific examples, a case is assumed in which the information processing system 2 provides a health support application to assist a user to lead a healthy life. The health support application accepts input operations related to life information on a user and provides the user with information useful for maintaining good health. The health support application is realized when the user executes a predetermined application on the terminal device 600 and communication is performed between the terminal device 600 and the information processing apparatus 200. The following screen examples of the health support application are displayed by the terminal output unit 660 based on the control by the terminal processing unit 640.

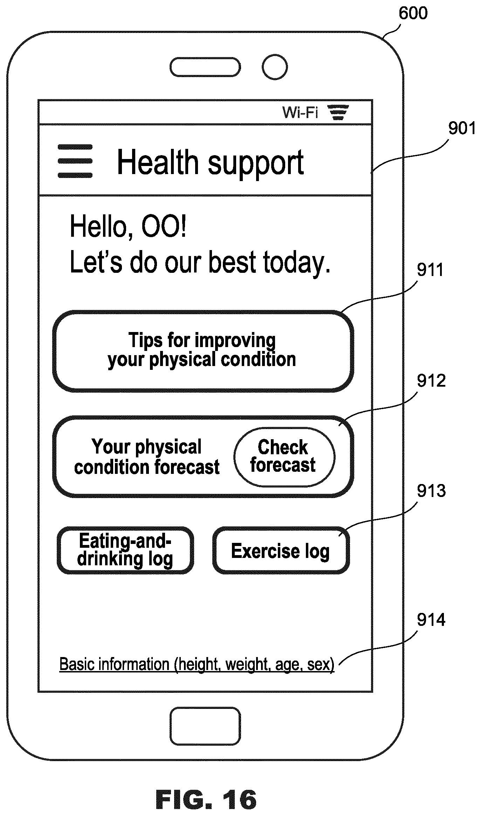

FIG. 16 is a first diagram showing a specific example of screen transition of the terminal device 600.

The drawing shows a menu screen 901 of the health support application. The menu screen 901 includes, for example, an improvement information display area 911, a score information display area 912 for displaying information on scores, life information record buttons 913 for performing processing such as performing input regarding life information and viewing a history, a basic information button 914 for checking and editing basic information, and the like.

The improvement information display area 911 displays, for example, information useful for improving the user's physical condition. If the improvement information display area 911 is operated, a transition is made to an improvement information display screen (not shown). The improvement information display screen may display predetermined information regardless of the user's condition. Also, information may be displayed according to score information that has been output in the past. For example, the content of previously acquired advice may be displayed so that the user can check the content of the advice again.

The score information display area 912 is a button for instructing the information processing apparatus 200 to execute output of score information. In this specific example, if the score information display area 912 is operated, the score information is acquired by the information processing apparatus 200 as described above, and the output information is transmitted to the terminal device 600. If the output information is received by the terminal device 600, a transition is made to a score display screen 904 (shown in the next drawing).

The life information record buttons 913 are buttons, for example, for inputting the time when the user ate or drank and the content thereof, the time when the user exercised and the content thereof, or the like. The user can input and record information on each element, or transmit the information to the information processing apparatus 200 for storage in the information processing apparatus 200, by operating the buttons corresponding to the elements.

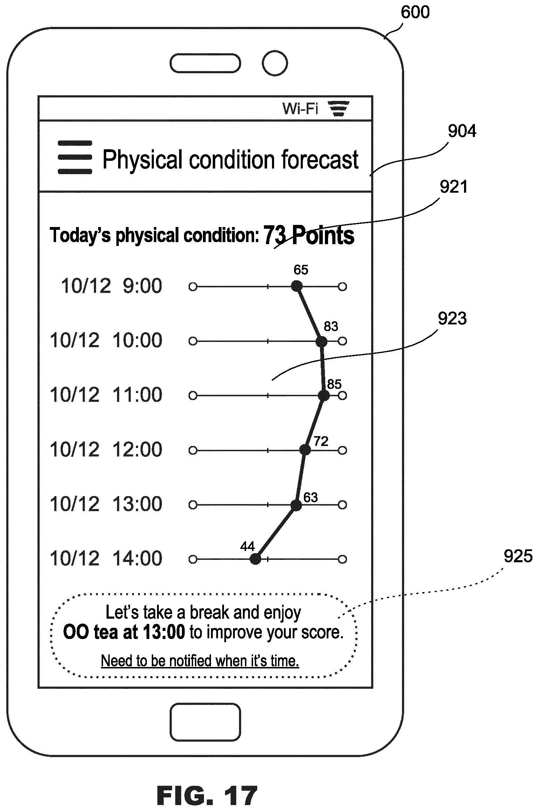

FIG. 17 is a second diagram showing a specific example of screen transition of the terminal device 600.

The drawing shows the score display screen 904 of the health support application. The score display screen 904 includes, for example, a score display area 921 that displays the overall score acquired, a prediction display area 923 that displays a prediction of future transition of the score, and an advice display area 925 that displays advice. The score display screen 904 allows the user to check the score regarding the physical condition.

The score display area 921 displays, for example, an overall score indicating the present score and the prediction results of the score in a predetermined period of time in the future, as one overall score. The overall score may be, for example, various values such as an average of the scores or a score at a predetermined point in time. The overall score allows the user to easily understand the level of his or her own physical condition.

Furthermore, the prediction display area 923 displays the transition of the score over a predetermined period of time in the future. The user can learn about the possibility of future changes in his or her physical condition by checking the prediction display area 923.

The advice display area 925 displays, for example, advice acquired by the output information acquiring unit 257. In the example shown in the drawing, based on the prediction result that the score will decrease around 14:00, the advice recommending that the user consume a beverage that is expected to decrease the stress level at the corresponding time is output. The display of such advice can give suggestions to the user regarding actions to enhance his or her physical condition and effectively motivate the user to improve his or her behavior.

As explained above, in Embodiment 2, it is possible to acquire a score indicating a physical condition of a user. It is possible to effectively make the user aware of the user's physical condition, by presenting the score to the user. This can effectively raise the user's awareness in order to maintain a healthy and active state, and to improve his or her behavior. If the user is kept in a good physical condition, the user's productivity can be improved and the society in which the user is involved can be revitalized.

The information processing apparatus 200 may be constituted by a single server, multiple servers that operate in cooperation with each other, or a computer or other device built into other equipment. It will be appreciated that the servers may be so-called cloud servers, ASP servers, or the like, and there is no limitation on the type thereof.

The processing in Embodiment 2 may be realized by software. The software may be distributed by software downloads or the like. Furthermore, the software may be distributed in a form where the software is stored in a recording medium such as an optical disk. The software that realizes the information processing apparatus 200 in this embodiment is the following sort of program. Specifically, this program is a program that is executed on a computer of the information processing apparatus 200, the program causing the computer of the information processing apparatus 200 capable of accessing a measurement information storage unit in which measurement information on a status of a living body based on measurement results of a bioelectric potential of a user at two or more points in time is stored, to function as: a biological information acquiring unit that acquires the measurement information from the measurement information storage unit; and a score acquiring unit that acquires score information containing a score indicating a present or future physical condition of the user, based on the measurement information.

In Embodiment 2, the processing unit 140 may not have the prediction information acquiring unit 145 and the future information output unit 147 among these units. In this case, scores may be acquired using measurement information based on measurement results actually measured using the measuring device 700 or the like. In this case, score information containing a score indicating the user's present physical condition can be acquired and output information based on that score information can be output.

### Others

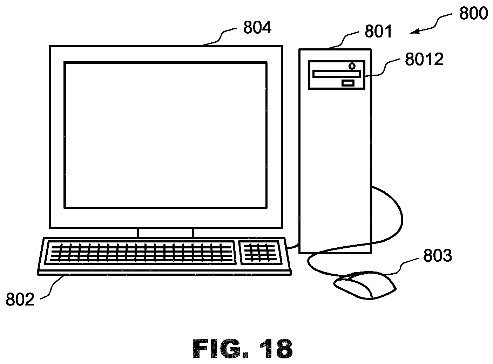

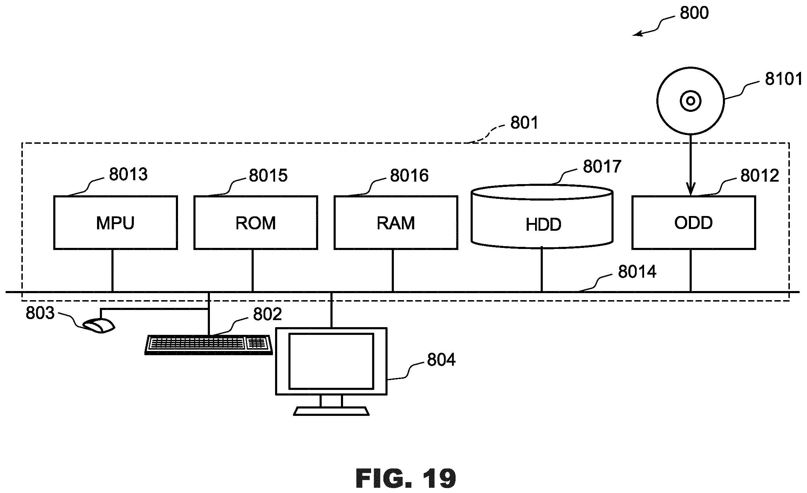

FIG. 18 is a schematic view of a computer system 800 in the foregoing embodiments. FIG. 19 is a block diagram of the computer system 800 in the embodiments.

These drawings show a configuration example of a computer that executes the programs described in this specification to realize the information processing apparatus and the like in the foregoing embodiments. The foregoing embodiments may be realized using computer hardware and a computer program executed thereon.

The computer system 800 includes a computer 801 including an optical disk drive, a keyboard 802, a mouse 803, and a monitor 804.

The computer 801 includes, in addition to the optical disk drive (ODD) 8012, an MPU 8013, a bus 8014 connected to the optical disk drive 8012 and the like, a ROM 8015 in which a program such as a boot up program is stored, a RAM 8016 that is connected to the MPU 8013 and is a memory in which a command of an application program is temporarily stored and a temporary storage area is provided, and a hard disk (HDD) 8017 in which programs such as an application program and a system program and data are stored. Although not shown, the computer 801 may further include a network card that provides connection to a LAN.

Each program for causing the computer system 800 to execute the functions of the information processing apparatus and the like in the foregoing embodiments may be stored in a optical disk 8101 that is inserted into the optical disk drive 8012, and be transmitted to the hard disk 8017. Alternatively, the program may be transmitted via a network (not shown) to the computer 801 and stored in the hard disk 8017. At the time of execution, the program is loaded into the RAM 8016. The program may be loaded from the optical disk 8101, or may be loaded directly from a network.

The program does not necessarily have to include, for example, an operating system (OS) or a third party program to cause the computer 801 to execute the functions of the information processing apparatus and the like in the foregoing embodiments. The program may only include a command portion to call an appropriate function (module) in a controlled mode and obtain desired results. The manner in which the computer system 800 operates is well known, and thus a detailed description thereof has been omitted.

It should be noted that, in the program, in a transmitting step of transmitting information, a receiving step of receiving information, or the like, processing that is performed only hardware such as processing that is performed by a modem or an interface card in the transmitting step (processing that can be performed only by hardware) is not included.

Furthermore, the computer that executes the program may be constituted by a single computer, or constituted by multiple computers. That is to say, centralized processing may be performed, or distributed processing may be performed.

Furthermore, in the foregoing embodiments, two or more constituent elements in one apparatus may be physically realized by one medium.

In the foregoing embodiments, each process (function) may be realized as centralized processing using a single apparatus (system), or may be realized as distributed processing using multiple apparatuses (in this case, the entire system constituted by multiple apparatuses that perform distributed processing may be regarded as one "apparatus").

Furthermore, in the foregoing embodiments, information transmission performed between constituent elements may be such that, for example, if two constituent elements for transmitting information are physically different from each other, the transmission is performed by one of the constituent elements outputting the information and the other constituent element accepting the information, or alternatively, if two constituent elements for transmitting information are physically the same, the transmission is performed by shifting from a processing phase corresponding to one of the constituent elements to a processing phase corresponding to the other constituent element.

Furthermore, in the foregoing embodiments, information related to the processing that is performed by each constituent element, for example, information that is to be accepted, acquired, selected, generated, transmitted, or received by each constituent element, information such as a threshold value, a numerical expression, or an address used by each constituent element in the processing and the like may be retained in an unshown recording medium temporarily or for a long period of time even if not specified in the description above. Furthermore, the information may be accumulated in the unshown recording medium by each constituent element or by an unshown accumulating unit. Furthermore, the information may be read from the unshown recording medium by each constituent element or by an unshown reading unit.

Furthermore, in the foregoing embodiments, if information used by each constituent element or the like, for example, information such as a threshold value, an address, or various setting values used by each constituent element in the processing may be changed by a user, the user may be or may not be allowed to change such information as appropriate even if not specified in the description above. If the user is allowed to change such information, the change may be realized by, for example, an unshown accepting unit that accepts a change instruction from the user and an unshown changing unit that changes information according to the change instruction. The unshown accepting unit may accept the change instruction, for example, by accepting information from an input device, by receiving information transmitted via a communication line, or by accepting information read from a predetermined recording medium.

The present invention is not limited to the embodiments set forth herein. Various modifications are possible within the scope of the invention.

An embodiment may be configured by combining constituent elements of the foregoing multiple embodiments or modified examples as appropriate. For example, the configuration of the foregoing embodiments is not limited to those described above, and constituent elements of the foregoing embodiments or modified examples may be replaced by or combined with constituent elements of other embodiments as appropriate. Some of the constituent elements or the functions may be omitted in the foregoing embodiments or modified examples.

### Industrial Applicability

As described above, the information processing apparatus according to the present invention makes it possible to acquire a score indicating a present or future physical condition of an individual user, thus rendering this apparatus useful as an information processing apparatus and the like.

### List of Reference Numerals

- 1, 2: Information processing system
- 100, 200: Information processing apparatus
- 110: Storage unit
- 111: Learning information storage unit
- 115: User information storage unit
- 120: Receiving unit
- 130: Accepting unit
- 140: Processing unit
- 143: Biological information acquiring unit
- 145: Prediction information acquiring unit
- 146: Pre-processing unit
- 147: Future information output unit
- 170: Transmitting unit
- 241: Evaluation information acquiring unit
- 242: Life information acquiring unit
- 249: Learning information acquiring unit
- 251: Score acquiring unit
- 257: Output information acquiring unit
- 260: Output unit
- 600: Terminal device
- 610: Terminal storage unit
- 620: Terminal receiving unit
- 630: Terminal accepting unit
- 640: Terminal processing unit
- 660: Terminal output unit
- 661: Display unit
- 670: Terminal transmitting unit
- 680: Sensor unit
- 700: Measuring device
- 702: Electrode unit
- 706: Measuring device output unit

## Claims

1. An information processing apparatus comprising:
a biological information acquiring unit that acquires measurement information on a status of a living body, based on measurement results of a bioelectric potential of a user at two or more points in time; and
a score acquiring unit that acquires score information containing a score indicating a present or future physical condition of the user, based on the measurement information.

2. The information processing apparatus according to claim 1, wherein the measurement information is time-series information.

3. The information processing apparatus according to claim 1, wherein the score information contains information indicating transition of the score.

4. The information processing apparatus according to claim 1, further comprising:
an output information acquiring unit that acquires output information on the score based on the score information; and
an output unit that outputs the output information.

5. The information processing apparatus according to claim 4, wherein the output information acquiring unit acquires a largest value of a score of score information that may be acquired for the user, and acquires output information corresponding to a relationship between the largest value and the score information acquired by the score acquiring unit.

6. The information processing apparatus according to claim 4, wherein the measurement information is information obtained by measuring a bioelectric potential of a user using a measuring device having two or more electrodes that are in contact with a body surface of the user.

7. The information processing apparatus according to claim 6, wherein the output information acquiring unit acquires output information for outputting a sound from the measuring device.

8. The information processing apparatus according to claim 4, wherein the output information acquiring unit acquires, as the output information, advice on eating-and-drinking behavior of a user to ensure that a score higher than the score of the score information acquired by the score acquiring unit is obtained in the future.

9. The information processing apparatus according to claim 1, wherein the score acquiring unit acquires the score information, based on information obtained by measuring at least one of a blood pressure, a pulse, a blood oxygen level, a body temperature, a respiratory rate, and an acceleration of a user.

10. The information processing apparatus according to claim 1, wherein the score acquiring unit acquires the score information, based on eating-and-drinking information on eating-and-drinking behavior of a user.

11. The information processing apparatus according to claim 1, wherein the score acquiring unit acquires future measurement information, based on input information on the measurement information, using learning information configured such that the input information is taken as input and the future measurement information is taken as output, and acquires the score information based on the acquired measurement information.

12. The information processing apparatus according to claim 1, wherein the score acquiring unit acquires information on the score, based on input information on the measurement information, using learning information configured such that the input information is taken as input and the information on the score is taken as output, and acquires the score information using the acquired information.

13. The information processing apparatus according to claim 12, wherein the score acquiring unit uses, as the learning information, learning information configured using input information containing measurement information obtained by measuring two or more users in the past.

14. The information processing apparatus according to claim 12, further comprising a learning information acquiring unit that acquires learning information obtained by performing re-learning using evaluation information corresponding to the score information acquired by the score acquiring unit and input information used to acquire the score information.

15. An information processing method performed using a measurement information storage unit in which measurement information on a status of a living body based on measurement results of a bioelectric potential of a user at two or more points in time is stored, comprising:
a biological information acquiring step of acquiring the measurement information from the measurement information storage unit; and
a score acquiring step of acquiring score information containing a score indicating a present or future physical condition of the user, based on the measurement information.

16. A program for causing a computer capable of accessing a measurement information storage unit in which measurement information on a status of a living body based on measurement results of a bioelectric potential of a user at two or more points in time is stored, to function as:
a biological information acquiring unit that acquires the measurement information from the measurement information storage unit; and
a score acquiring unit that acquires score information containing a score indicating a present or future physical condition of the user, based on the measurement information.
